(19) **Europäisches Patentamt European Patent Office Office européen des brevets**

(11) **EP 4 471 060 A1**

(12) **EUROPEAN PATENT APPLICATION**
published in accordance with Art. 153(4) EPC

(43) Date of publication:
04.12.2024 Bulletin 2024/49

(21) Application number: 23746433.4

(22) Date of filing: 29.01.2023

(51) International Patent Classification (IPC):
C07K 16/24 (2006.01)    A61K 47/68 (2017.01)
A61P 35/00 (2006.01)    A61P 29/00 (2006.01)

(52) Cooperative Patent Classification (CPC):
A61K 47/68; A61P 29/00; A61P 35/00; C07K 16/24

(86) International application number:
PCT/CN2023/073670

(87) International publication number:
WO 2023/143556 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.01.2022 CN 202210112310

(71) Applicant: Mabwell (Shanghai) Bioscience Co.,
Ltd.
Shanghai 201210 (CN)

(72) Inventors:
• ZENG, Dadi
  Shanghai 201210 (CN)
• LIU, Datao
  Shanghai 201210 (CN)
• GUI, Xun
  Shanghai 201212 (CN)

• WANG, Rongjuan
  Shanghai 201210 (CN)
• LU, Weining
  Shanghai 201210 (CN)
• JIAO, Shasha
  Shanghai 201210 (CN)
• ZHANG, Chang
  Shanghai 201210 (CN)
• WANG, Shuang
  Shanghai 201210 (CN)

(74) Representative: Schiweck Weinzierl Koch
Patentanwälte Partnerschaft mbB
Ganghoferstraße 68 B
80339 München (DE)

Remarks:
The complete document including Reference
Table(s) and the Sequence Listing(s) can be
downloaded from the EPO website

(54) **ANTI-INTERLEUKIN-11 ANTIBODY AND USE THEREOF**

(57) Disclosed is an anti-interleukin 11 (IL-11) antibody or a fragment thereof, which has high affinity for antigen IL-11, can block the formation of an IL-11/IL-11Rα/GP130 complex and inhibit the activation of the STAT3 signal pathway. Therefore, the anti-interleukin 11 antibody or the fragment thereof can effectively neutralize the activity of IL-11, and can be used for preventing, treating and/or improving diseases or conditions related to IL-11 expression (including overexpression).

EP 4 471 060 A1

**Description**

<u>CROSS-REFERENCE TO RELATED APPLICATION(S)</u>

The present patent application claims the benefit of priority to Chinese Patent Application for Invention No. CN202210112310.7 filed on 29 January 2022, which is incorporated herein by reference in its entirety for all purposes.

<u>TECHNICAL FIELD</u>

The invention belongs to the field of biomedicine, and relates to an antibody or antigen binding fragment thereof against Interleukin-11 (IL-11) and applications of the antibody or antigen binding fragment thereof.

<u>BACKGROUND OF THE INVENTION</u>

IL-11, discovered in 1990, belongs to the members of the IL-6 family, and comprises 178 amino acids and has a molecular weight of approximately 19 kDa. IL-11 can be expressed by various cells including leucocytes, fibroblasts, epithelial cells, and the like, and its expression is regulated by various cytokines, e.g., TGF-$\beta$, IL-1$\beta$, IL-22, IL-17F, IFN-$\gamma$, TNF-$\alpha$, COX-2, etc. IL-11 activates downstream signaling pathways by binding to IL-11 receptor alpha subunit (IL-11R$\alpha$) and in turn forming a 2:2:2 (IL-11/IL-11R$\alpha$/GP130) complex with the glycoprotein beta receptor subunit (GP130).

The most clearly studied physiological function of IL-11 is to promote the expansion of megakaryocytes, and thus, the production of platelets. In addition to hematopoietic function, another major physiological function of IL-11 is to promote osteogenesis (osteoblasts constitutively express IL-11 and IL-11R$\alpha$). IL-11 is a downstream response gene of TGF-$\beta$, and its expression is regulated by TGF-$\beta$. It has been reported that IL-11 can stimulate the production of erythrocytes, promote the differentiation of macrophages and NK cells, regulate the release of Th1/Th2-related cytokines and in turn reduce tissue damage caused by inflammation.

Researchers reported that the expression of IL-11 was up-regulated in various tumors, including melanoma, breast cancer, colorectal cancer, non-small cell lung cancer, etc. In mouse models, tumor-infiltrating macrophages as well as T cells, in addition to tumor cells, were found to express IL-11. In human colorectal cancer tissues, tumor-associated fibroblasts were the major source of expression of IL-11. IL-11 was found to involve in tumor-promoting processes at multiple levels through activating STAT3. Furthermore, the expression of IL-11 was directly related to the prognosis of various tumors. In breast cancer, up-regulation of IL-11 could further promote bone metastasis of the tumor. The incidence of colorectal cancer in mice with IL-11R$\alpha$ gene knockout was significantly lower than that in wild-type mice.

IL-11 is also known to involve in renal and cardiac fibrosis; and a study found that the expression of IL-11 was up-regulated in the lungs of patients suffering from Idiopathic Pulmonary Fibrosis (IPF), and correlated with the severity of the disease in lungs. Studies in vitro and in animal models on IL-11 and its receptors suggest that IL-11 can be considered as a drug target for a variety of fibrosis-related diseases, including pulmonary fibrosis.

There have been a number of studies on anti-IL-11 monoclonal antibodies in prior arts; however, the signaling mechanism by which anti-IL-11 monoclonal antibodies function and the downstream therapeutic effects of anti-IL-11 monoclonal antibodies remain to be further investigated. Especially, IL-11 has various biological activities and participates in various physiological functions, and screening and obtaining new monoclonal antibodies which are capable of specifically binding to sites on IL-11 which are to be bound by IL-11R$\alpha$ and/or GP130 and inhibiting the formation of IL-11/IL-11R$\alpha$/GP130 complex and the activation of STAT3, has important significance for treating diseases related to expression or over-expression of IL-11 and to abnormal activation of STAT3.

<u>SUMMARY OF THE INVENTION</u>

In view of the above-mentioned problems, the object of the present disclosure is to provide a novel antibody or antigen binding fragment thereof against IL-11, in particular human IL-11. Compared with existing anti-IL-11 antibodies, the antibody provided by the present disclosure has higher affinity for the antigen IL-11, can block the formation of an IL-11/IL-11R$\alpha$/GP130 complex and inhibit the activation of a STAT3 signaling pathway with higher activity, thereby being capable of neutralizing the activity of IL-11 more effectively.

The present disclosure provides technical solutions as follows.

In one aspect, the present disclosure provides an antibody or fragment thereof capable of binding to antigen IL-11, particularly human IL-11, with high affinity. According to particular embodiments of the present disclosure, the present disclosure provides antibodies as follows: murine antibodies obtained by using human IL-11 or murine II,-11 as an immunogen; humanized antibodies obtained through humanization of the murine antibodies; and antibodies obtained through sequence optimization of the humanized antibodies by using yeast display technique.

Specifically, the present disclosure provides an antibody or fragment thereof comprising:

a heavy chain variable region (VH) which comprises Complementarity Determining Regions (CDRs), i.e. H-CDR1, H-CDR2 and H-CDR3; and, a light chain variable region (VL) which comprises CDRs, i.e. L-CDR1, L-CDR2 and L-CDR3.

In some particular embodiments, in the antibody or fragment thereof:

(1)

the heavy chain variable region comprises heavy chain CDR1 (H-CDR1), heavy chain CDR2 (H-CDR2) and heavy chain CDR3 (H-CDR3) derived from the heavy chain variable region comprising the amino acid sequence as follows:

an amino acid sequence as shown in SEQ ID NO.7, SEQ ID NO.11, SEQ ID NO.15, SEQ ID NO.16, SEQ ID NO.17, SEQ ID NO.18, SEQ ID NO.19, SEQ ID NO.20, SEQ ID NO 21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31, SEQ ID NO.32 or SEQ ID NO.33; and

the light chain variable region comprises light chain CDR 1 (L-CDR1), light chain CDR 2 (L-CDR2) and light chain CDR 3 (L-CDR3) derived from the light chain variable region comprising the amino acid sequence as follows:

an amino acid sequence as shown in SEQ ID NO.8, SEQ ID NO.12, SEQ ID NO.34, SEQ ID NO.35, SEQ ID NO.36, SEQ ID NO.37, SEQ ID NO.38, SEQ ID NO.39, SEQ ID NO.40, SEQ ID NO.41, SEQ ID NO.42, SEQ ID NO.43, SEQ ID NO.44, SEQ ID NO.45 or SEQ ID NO.46;

alternatively,

(2)

the heavy chain variable region comprises heavy chain CDR1 (H-CDR1), heavy chain CDR2 (H-CDR2) and heavy chain CDR3 (H-CDR3) derived from the heavy chain variable region comprising the amino acid sequence as follows:

an amino acid sequence as shown in SEQ ID NO.5, SEQ ID NO.9, SEQ ID NO.49, SEQ ID NO.50, SEQ ID NO.51, SEQ ID NO.52, SEQ ID NO.53, SEQ ID NO.54, SEQ ID NO.55, SEQ ID NO.56, SEQ ID NO.57, SEQ ID NO.58, SEQ ID NO.59, SEQ ID NO.60, SEQ ID NO.61, SEQ ID NO.62, SEQ ID NO.63, SEQ ID NO.64, SEQ ID NO.65, SEQ ID NO.66, SEQ ID NO.67 or SEQ ID NO.68; and

the light chain variable region comprises light chain CDR 1 (L-CDR1), light chain CDR 2 (L-CDR2) and light chain CDR 3 (L-CDR3) derived from the light chain variable region comprising the amino acid sequence as follows:

an amino acid sequence as shown in SEQ ID NO.6, SEQ ID NO.10, SEQ ID NO.69, SEQ ID NO.70, SEQ ID NO.71, SEQ ID NO.72, SEQ ID NO.73, SEQ ID NO.74, SEQ ID NO.75, SEQ ID NO.76, SEQ ID NO.77, SEQ ID NO.78, SEQ ID NO.79, SEQ ID NO.80, SEQ ID NO.81, SEQ ID NO.82, SEQ ID NO.83, SEQ ID NO.84, SEQ ID NO.85, SEQ ID NO.86 or SEQ ID NO.87.

The amino acid sequences in the above groups (1) and (2) are the amino acid sequences of heavy chain variable regions or light chain variable regions comprised in the anti-IL-11 antibodies provided by the present disclosure, respectively. Using schemes well known in the art for the definition of complementarity determining regions in the heavy or light chain variable region of an antibody (e.g., Chothia, Kabat, IMGT, Contact, etc.), one person skilled in the art can readily determine the heavy and light chain CDRs contained in each sequence. According to particular embodiments of the present disclosure, the CDRs in the sequences of variable regions are defined using the Kabat numbering.

Preferably, in the antibody or fragment thereof provided by the present disclosure, the heavy chain variable region and the light chain variable region comprise a heavy chain CDR1, a heavy chain CDR2 and a heavy chain CDR3, and a light chain CDR1, a light chain CDR2 and a light chain CDR3, respectively, from the heavy chain variable region and the light chain variable region as shown in the following combinations of amino acid sequences:

(1)

(1-1) SEQ ID NO.7 + SEQ ID NO.8;
(1-2) SEQ ID NO.11 + SEQ ID NO.12;
(1-3) SEQ ID NO.15 + SEQ ID NO.12;
(1-4) SEQ ID NO.16 + SEQ ID NO.12;
(1-5) SEQ ID NO.17 + SEQ ID NO.12;
(1-6) SEQ ID NO.18 + SEQ ID NO.12;
(1-7) SEQ ID NO.15 + SEQ ID NO.34;
(1-8) SEQ ID NO.15 + SEQ ID NO.35;
(1-9) SEQ ID NO.15 + SEQ ID NO.36;

(1-10) SEQ ID NO.15 + SEQ ID NO.37;
(1-11) SEQ ID NO.15 + SEQ ID NO.38;
(1-12) SEQ ID NO.15 + SEQ ID NO.39;
(1-13) SEQ ID NO.15 + SEQ ID NO.40;
(1-14) SEQ ID NO.15 + SEQ ID NO.41;
(1-15) SEQ ID NO.15 + SEQ ID NO.42;
(1-16) SEQ ID NO.19 + SEQ ID NO.40;
(1-17) SEQ ID NO.20 + SEQ ID NO.40;
(1-18) SEQ ID NO.21 + SEQ ID NO.40;
(1-19) SEQ ID NO.22 + SEQ ID NO.40;
(1-20) SEQ ID NO.23 + SEQ ID NO.40;
(1-21) SEQ ID NO.15 + SEQ ID NO.43;
(1-22) SEQ ID NO.15 + SEQ ID NO.44;
(1-23) SEQ ID NO.15 + SEQ ID NO.45;
(1-24) SEQ ID NO.15 + SEQ ID NO.46;
(1-25) SEQ ID NO.23 + SEQ ID NO.43;
(1-26) SEQ ID NO.23 + SEQ ID NO.44;
(1-27) SEQ ID NO.23 + SEQ ID NO.45;
(1-28) SEQ ID NO.23 + SEQ ID NO.46;
(1-29) SEQ ID NO.24 + SEQ ID NO.44;
(1-30) SEQ ID NO.25 + SEQ ID NO.44;
(1-31) SEQ ID NO.26 + SEQ ID NO.44;
(1-32) SEQ ID NO.27 + SEQ ID NO.44;
(1-33) SEQ ID NO.28 + SEQ ID NO.44;
(1-34) SEQ ID NO.29 + SEQ ID NO.44;
(1-35) SEQ ID NO.30 + SEQ ID NO.44;
(1-36) SEQ ID NO.31 + SEQ ID NO.44;
(1-37) SEQ ID NO.32 + SEQ ID NO.44;
(1-38) SEQ ID NO.33 + SEQ ID NO.44;
alternatively,
(2)

(2-1) SEQ ID NO.5 + SEQ ID NO.6;
(2-2) SEQ ID NO.9 + SEQ ID NO.10;
(2-3) SEQ ID NO.9 + SEQ ID NO.69;
(2-4) SEQ ID NO.9 + SEQ ID NO.70;
(2-5) SEQ ID NO.9 + SEQ ID NO.71;
(2-6) SEQ ID NO.9 + SEQ ID NO.72;
(2-7) SEQ ID NO.9 + SEQ ID NO.73;
(2-8) SEQ ID NO.9 + SEQ ID NO.74;
(2-9) SEQ ID NO.9 + SEQ ID NO.76;
(2-10) SEQ ID NO.9 + SEQ ID NO.77;
(2-11) SEQ ID NO.9 + SEQ ID NO.78;
(2-12) SEQ ID NO.9 + SEQ ID NO.79;
(2-13) SEQ ID NO.9 + SEQ ID NO.80;
(2-14) SEQ ID NO.9 + SEQ ID NO.81;
(2-15) SEQ ID NO.49 + SEQ ID NO.10;
(2-16) SEQ ID NO.50 + SEQ ID NO.10;
(2-17) SEQ ID NO.49 + SEQ ID NO.75;
(2-18) SEQ ID NO.49 + SEQ ID NO.82;
(2-19) SEQ ID NO.49 + SEQ ID NO.83;
(2-20) SEQ ID NO.49 + SEQ ID NO.77;
(2-21) SEQ ID NO.49 + SEQ ID NO.79;
(2-22) SEQ ID NO.50 + SEQ ID NO.77;
(2-23) SEQ ID NO.50 + SEQ ID NO.79;
(2-24) SEQ ID NO.53 + SEQ ID NO.77;
(2-25) SEQ ID NO.9 + SEQ ID NO.84;
(2-26) SEQ ID NO.9 + SEQ ID NO.85;

(2-27) SEQ ID NO.51 + SEQ ID NO.77;
(2-28) SEQ ID NO.52 + SEQ ID NO.77;
(2-29) SEQ ID NO.54 + SEQ ID NO.77;
(2-30) SEQ ID NO.9 + SEQ ID NO.86;
(2-31) SEQ ID NO.52 + SEQ ID NO.86;
(2-32) SEQ ID NO.55 + SEQ ID NO.87;
(2-33) SEQ ID NO.56 + SEQ ID NO.87;
(2-34) SEQ ID NO.57 + SEQ ID NO.87;
(2-35) SEQ ID NO.51 + SEQ ID NO.87;
(2-36) SEQ ID NO.54 + SEQ ID NO.87;
(2-37) SEQ ID NO.58 + SEQ ID NO.87;
(2-38) SEQ ID NO.59 + SEQ ID NO.87;
(2-39) SEQ ID NO.60 + SEQ ID NO.87;
(2-40) SEQ ID NO.61 + SEQ ID NO.87;
(2-41) SEQ ID NO.62 + SEQ ID NO.87;
(2-42) SEQ ID NO.63 + SEQ ID NO.87;
(2-43) SEQ ID NO.64 + SEQ ID NO.87;
(2-44) SEQ ID NO.65 + SEQ ID NO.87;
(2-45) SEQ ID NO.66 + SEQ ID NO.87;
(2-46) SEQ ID NO.67 + SEQ ID NO.87;
(2-47) SEQ ID NO.68 + SEQ ID NO.87.

As described above, the CDRs in each sequence of variable region in the above combinations can be defined using the Kabat numbering; see those shown in the Examples of the present disclosure (in particular, Table 3 and Table 6); and in the above combinations, the combination of CDRs in the heavy chain variable regions and the light chain variable regions are contained in the antibody or the fragment thereof provided by the present disclosure.

Accordingly, in the antibody or fragment thereof provided by the present disclosure, the heavy chain variable region and the light chain variable region comprise a combination of heavy chain CDRs and light chain CDRs (H-CDR1, H-CDR2, and H-CDR3; and, L-CDR1, L-CDR2, and L-CDR3) selected from the group consisting of

(1)

(1-1) H-CDR1, H-CDR2, and H-CDR3 comprising the amino acid sequences as shown in SEQ ID NO.94, SEQ ID NO.95, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-2) H-CDR1, H-CDR2, and H-CDR3 comprising the amino acid sequences as shown in SEQ ID NO.94, SEQ ID NO. 100, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-3) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-4) H-CDR1, H-CDR2, and H-CDR3 comprising the amino acid sequences as shown in SEQ ID NO.94, SEQ ID NO.102, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-5) H-CDR1, H-CDR2, and H-CDR3 comprising the amino acid sequences as shown in SEQ ID NO.94, SEQ ID NO.103, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-6) H-CDR1, H-CDR2, and H-CDR3 comprising the amino acid sequences as shown in SEQ ID NO.94, SEQ ID NO.104, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-7) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.115, SEQ ID NO.98, and SEQ ID NO.99 successively;

(1-8) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.116, SEQ ID NO.98, and SEQ ID NO.99 successively;

(1-9) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.117, SEQ ID NO.98, SEQ ID NO.99 successively;

(1-10) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 118, SEQ ID NO.98, and SEQ ID NO.99 successively;

(1-11) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 119, SEQ ID NO.98, and SEQ ID NO.99 successively;

(1-12) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 120, SEQ ID NO.98, and SEQ ID NO.99 successively;

(1-13) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-14) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 122, SEQ ID NO.98, and SEQ ID NO.99 successively;

(1-15) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 123, SEQ ID NO.98, and SEQ ID NO.99 successively;

(1-16) H-CDR1, H-CDR2, and H-CDR3 comprising the amino acid sequences as shown in SEQ ID NO.105, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-17) H-CDR1, H-CDR2, and H-CDR3 comprising the amino acid sequences as shown in SEQ ID NO.94, SEQ ID NO.106, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-18) H-CDR1, H-CDR2, and H-CDR3 comprising the amino acid sequences as shown in SEQ ID NO.107, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-19) H-CDR1, H-CDR2, and H-CDR3 comprising the amino acid sequences as shown in SEQ ID NO.94, SEQ ID NO.108, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-20) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO. 121, SEQ ID NO.98, and SEQ ID NO.99 successively;

(1-21) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.124, and SEQ ID NO.99 successively;

(1-22) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.125, and SEQ ID NO.99 successively;

(1-23) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.126, and SEQ ID NO.99 successively;

(1-24) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.127, and SEQ ID NO.99 successively;

(1-25) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.124, and SEQ ID NO.99 successively;

(1-26) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.125, and SEQ ID NO.99 successively;

(1-27) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.126, and SEQ ID NO.99 successively;

(1-28) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.127, and SEQ ID NO.99 successively;

(1-29) H-CDR1, H-CDR2, and H-CDR3 comprising the amino acid sequences as shown in SEQ ID NO.94, SEQ ID NO.110, and SEQ ID NO.96 successively; and, L-CDR1, L-CDR2, and L-CDR3 comprising the amino acid sequences as shown in SEQ ID NO.121, SEQ ID NO.125, and SEQ ID NO.99 successively;

(1-30) H-CDR1, H-CDR2, and H-CDR3 comprising the amino acid sequences as shown in SEQ ID NO.94, SEQ ID NO.101, and SEQ ID NO.111 successively; and, L-CDR1, L-CDR2, and L-CDR3 comprising the amino acid sequences as shown in SEQ ID NO.121, SEQ ID NO.125, and SEQ ID NO.99 successively;

(1-31) H-CDR1, H-CDR2, and H-CDR3 comprising the amino acid sequences as shown in SEQ ID NO.94, SEQ ID NO.101, and SEQ ID NO.112 successively; and, L-CDR1, L-CDR2, and L-CDR3 comprising the amino acid sequences as shown in SEQ ID NO.121, SEQ ID NO.125, and SEQ ID NO.99 successively;

(1-32) H-CDR1, H-CDR2, and H-CDR3 comprising the amino acid sequences as shown in SEQ ID NO.94, SEQ ID NO.101, and SEQ ID NO.113 successively; and, L-CDR1, L-CDR2, and L-CDR3 comprising the amino acid sequences as shown in SEQ ID NO.121, SEQ ID NO.125, and SEQ ID NO.99 successively; and

(1-33) H-CDR1, H-CDR2, and H-CDR3 comprising the amino acid sequences as shown in SEQ ID NO.94, SEQ ID NO.101, and SEQ ID NO.114 successively; and, L-CDR1, L-CDR2, and L-CDR3 comprising the amino acid sequences as shown in SEQ ID NO.121, SEQ ID NO.125, and SEQ ID NO.99 successively;

alternatively,

(2)

(2-1) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.92, and SEQ ID NO.93 successively;

(2-2) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.146, SEQ ID NO.92, and SEQ ID NO.93 successively;

(2-3) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.147, SEQ ID NO.92, and SEQ ID NO.93 successively;

(2-4) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.148, SEQ ID NO.92, and SEQ ID NO.93 successively;

(2-5) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.149, SEQ ID NO.92, and SEQ ID NO.93 successively;

(2-6) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.150, SEQ ID NO.92, and SEQ ID NO.93 successively;

(2-7) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.151, SEQ ID NO.92, and SEQ ID NO.93 successively;

(2-8) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.153, SEQ ID NO.92, and SEQ ID NO.93 successively;

(2-9) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.154, SEQ ID NO.92, and SEQ ID NO.93 successively;

(2-10) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO. 155, SEQ ID NO.92, and SEQ ID NO.93 successively;

(2-11) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO. 156, and SEQ ID NO.93 successively;

(2-12) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO. 157, and SEQ ID NO.93 successively;

(2-13) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO. 158, and SEQ ID NO.93 successively;

(2-14) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO.92, and SEQ ID NO.93 successively;

(2-15) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO.91, SEQ ID NO.92, and SEQ ID NO.93 successively;

(2-16) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO. 152, SEQ ID NO.92, and SEQ ID NO.93 successively;

(2-17) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO. 159, and SEQ ID NO.93 successively;

(2-18) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO. 160, and SEQ ID NO.93 successively;

(2-19) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO. 154, SEQ ID NO.92, and SEQ ID NO.93 successively;

(2-20) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO. 156, and SEQ ID NO.93 successively;

(2-21) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO. 154, SEQ ID NO.92, and SEQ ID NO.93 successively;

(2-22) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO.91, SEQ ID NO. 156, and SEQ ID NO.93 successively;

(2-23) H-CDR1, H-CDR2, and H-CDR3 comprising the amino acid sequence shown in sequence in SEQ ID NO.88, SEQ ID NO.89, and SEQ ID NO.134 successively; and, L-CDR1, L-CDR2, and L-CDR3 comprising the amino acid sequences as shown in SEQ ID NO.154, SEQ ID NO.92, and SEQ ID NO.93 successively;

(2-24) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO. 161, and SEQ ID NO.93 successively;

(2-25) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.162, SEQ ID NO.156, and SEQ ID NO.93 successively;

(2-26) H-CDR1, H-CDR2, and H-CDR3 comprising the amino acid sequences as shown in SEQ ID NO.132, SEQ ID NO.89, and SEQ ID NO.90 successively; and, L-CDR1, L-CDR2, and L-CDR3 comprising the amino acid sequences as shown in SEQ ID NO. 154, SEQ ID NO.92, and SEQ ID NO.93 successively;

(2-27) H-CDR1, H-CDR2, and H-CDR3 comprising the amino acid sequences as shown in SEQ ID NO.133, SEQ ID NO.89, SEQ ID NO.90 successively; and, L-CDR1, L-CDR2, and L-CDR3 comprising the amino acid sequences as shown in SEQ ID NO.154, SEQ ID NO.92, and SEQ ID NO.93 successively;

(2-28) H-CDR1, H-CDR2, and H-CDR3 comprising the amino acid sequences as shown in SEQ ID NO.135, SEQ ID NO.89, and SEQ ID NO.90 successively; and, L-CDR1, L-CDR2, and L-CDR3 comprising the amino acid sequences as shown in SEQ ID NO. 154, SEQ ID NO.92, and SEQ ID NO.93 successively;

(2-29) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.162, SEQ ID NO.160, and SEQ ID NO.93 successively;

(2-30) H-CDR1, H-CDR2, and H-CDR3 comprising the amino acid sequences as shown in SEQ ID NO.133, SEQ ID NO.89, and SEQ ID NO.90 successively; and, L-CDR1, L-CDR2, and L-CDR3 comprising the amino acid sequences as shown in SEQ ID NO.162, SEQ ID NO.160, and SEQ ID NO.93 successively;

(2-31) H-CDR1, H-CDR2, and H-CDR3 comprising the amino acid sequences as shown in SEQ ID NO.136, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-32) H-CDR1, H-CDR2, and H-CDR3 comprising the amino acid sequences as shown in SEQ ID NO.137, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-33) H-CDR1, H-CDR2, and H-CDR3 comprising the amino acid sequences as shown in SEQ ID NO.138, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-34) H-CDR1, H-CDR2, and H-CDR3 comprising the amino acid sequences as shown in SEQ ID NO.132, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-35) H-CDR1, H-CDR2, and H-CDR3 comprising the amino acid sequences as shown in SEQ ID NO.135, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-36) H-CDR1, H-CDR2, and H-CDR3 comprising the amino acid sequences as shown in SEQ ID NO.139, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-37) H-CDR1, H-CDR2, and H-CDR3 comprising the amino acid sequences as shown in SEQ ID NO.140, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-38) H-CDR1, H-CDR2, and H-CDR3 comprising the amino acid sequences as shown in SEQ ID NO.141, SEQ ID NO.89, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-39) H-CDR1, H-CDR2, and H-CDR3 comprising the amino acid sequences as shown in SEQ ID NO.142, SEQ ID NO.89, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-40) H-CDR1, H-CDR2, and H-CDR3 comprising the amino acid sequences as shown in SEQ ID NO.135, SEQ ID NO.143, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-41) H-CDR1, H-CDR2, and H-CDR3 comprising the amino acid sequences as shown in SEQ ID NO.135, SEQ ID NO.144, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-42) H-CDR1, H-CDR2, and H-CDR3 comprising the amino acid sequences as shown in SEQ ID NO.139, SEQ ID NO.144, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-43) H-CDR1, H-CDR2, and H-CDR3 comprising the amino acid sequences as shown in SEQ ID NO.139, SEQ ID NO.143, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively; and

(2-44) H-CDR1, H-CDR2, and H-CDR3 comprising the amino acid sequences as shown in SEQ ID NO.139, SEQ ID NO.145, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively.

Preferably, the antibody or fragment thereof provided by the present disclosure is an anti-interleukin-11 (IL-11) antibody or fragment thereof. Preferably, the IL-11 is human IL-11 (hIL-11). An exemplary amino acid sequence of human IL-11 is shown in GenBank Accession No.: AAH 12506.1.

Preferably, the anti-IL-11 antibody or fragment thereof in the present disclosure comprises at least a heavy chain variable region and a light chain variable region, both comprising CDRs as above and interspersed Framework Regions (FRs), which regions are arranged as follows: FR1-CDR1-FR2-CDR2-FR3 -CDR3 -FR4.

Further preferably, in the anti-IL-11 antibody or fragment thereof provided by the present disclosure:

(1)

the heavy chain variable region comprises an amino acid sequence selected from the group consisting of:
the amino acid sequence as shown in SEQ ID NO.7, SEQ ID NO.11, SEQ ID NO.15, SEQ ID NO.16, SEQ ID NO.17, SEQ ID NO.18, SEQ ID NO.19, SEQ ID NO.20, SEQ ID NO 21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31, SEQ ID NO.32, or SEQ ID NO.33, or an amino acid sequences having at least 75% identity to the amino acid sequence; and/or
the light chain variable region comprises an amino acid sequence selected from the group consisting of:
the amino acid sequence as shown in SEQ ID NO.8, SEQ ID NO.12, SEQ ID NO.34, SEQ ID NO.35, SEQ ID NO.36, SEQ ID NO.37, SEQ ID NO.38, SEQ ID NO.39, SEQ ID NO.40, SEQ ID NO.41, SEQ ID NO.42, SEQ ID NO.43, SEQ ID NO.44, SEQ ID NO.45, or SEQ ID NO.46, or an amino acid sequences having at least 75% identity to the amino acid sequence;
alternatively,

(2)

the heavy chain variable region comprises an amino acid sequence selected from the group consisting of: the amino acid sequence as shown in SEQ ID NO.5, SEQ ID NO.9, SEQ ID NO.49, SEQ ID NO.50, SEQ ID NO.51, SEQ ID NO.52, SEQ ID NO.53, SEQ ID NO.54, SEQ ID NO.55, SEQ ID NO.56, SEQ ID NO.57, SEQ ID NO.58, SEQ ID NO.59, SEQ ID NO.60, SEQ ID NO.61, SEQ ID NO.62, SEQ ID NO.63, SEQ ID NO.64, SEQ ID NO.65, SEQ ID NO.66, SEQ ID NO.67, or SEQ ID NO.68, or an amino acid sequences having at least 75% identity to the amino acid sequence; and/or

the light chain variable region comprises an amino acid sequence selected from the group consisting of: the amino acid sequence as shown in SEQ ID NO.6, SEQ ID NO.10, SEQ ID NO.69, SEQ ID NO.70, SEQ ID NO.71, SEQ ID NO.72, SEQ ID NO.73, SEQ ID NO.74, SEQ ID NO.75, SEQ ID NO.76, SEQ ID NO.77, SEQ ID NO.78, SEQ ID NO.79, SEQ ID NO.80, SEQ ID NO.81, SEQ ID NO.82, SEQ ID NO.83, SEQ ID NO.84, SEQ ID NO.85, SEQ ID NO.86, or SEQ ID NO.87, or an amino acid sequences having at least 75% identity to the amino acid sequence.

The up to 25% difference in an amino acid sequence due to the "at least 75% identity" may be present in any framework region in the heavy chain variable region or the light chain variable region, or, may be present in any domain or sequence in the antibody or fragment thereof according to the present disclosure other than the heavy chain variable region and the light chain variable region. The difference may be resulted from amino acid deletion, addition or substitution at any position, and the substitution may be conservative substitution or non-conservative substitution. The "at least 75% identity" encompasses any percent identity between 75% identity and 100% identity, e.g., 75%, 80%, 85%, 90%, even 91%, 92%, 93%, 94%, 95%, 96%, 97%, 98%, 99%, or even 100% identity.

According to particular embodiments of the present disclosure, in the anti-IL-11 antibody or fragment thereof provided by the present disclosure, the heavy chain variable region and the light chain variable region comprise a combination of amino acid sequences selected from the group consisting of:

(1)

(1-1) the amino acid sequence as shown in SEQ ID NO.7, or an amino acid sequence having at least 75% identity to the amino acid sequence; and, the amino acid sequence as shown in SEQ ID NO.8, or an amino acid sequence having at least 75% identity to the amino acid sequence;

(1-2) the amino acid sequence as shown in SEQ ID NO. 11, or an amino acid sequence having at least 75% identity to the amino acid sequence; and, the amino acid sequence as shown in SEQ ID NO. 12, or an amino acid sequence having at least 75% identity to the amino acid sequence;

(1-3) the amino acid sequence as shown in SEQ ID NO.15, or an amino acid sequence having at least 75% identity to the amino acid sequence; and, the amino acid sequence as shown in SEQ ID NO. 12, or an amino acid sequence having at least 75% identity to the amino acid sequence;

(1-4) the amino acid sequence as shown in SEQ ID NO.16, or an amino acid sequence having at least 75% identity to the amino acid sequence; and, the amino acid sequence as shown in SEQ ID NO.12, or an amino acid sequence having at least 75% identity to the amino acid sequence;

(1-5) the amino acid sequence as shown in SEQ ID NO.17, or an amino acid sequence having at least 75% identity to the amino acid sequence; and, the amino acid sequence as shown in SEQ ID NO.12, or an amino acid sequence having at least 75% identity to the amino acid sequence;

(1-6) the amino acid sequence as shown in SEQ ID NO.18, or an amino acid sequence having at least 75% identity to the amino acid sequence; and, the amino acid sequence as shown in SEQ ID NO.12, or an amino acid sequence having at least 75% identity to the amino acid sequence;

(1-7) the amino acid sequence as shown in SEQ ID NO.15, or an amino acid sequence having at least 75% identity to the amino acid sequence; and, the amino acid sequence as shown in SEQ ID NO.34, or an amino acid sequence having at least 75% identity to the amino acid sequence;

(1-8) the amino acid sequence as shown in SEQ ID NO.15, or an amino acid sequence having at least 75% identity to the amino acid sequence; and, the amino acid sequence as shown in SEQ ID NO.35, or an amino acid sequence having at least 75% identity to the amino acid sequence;

(1-9) the amino acid sequence as shown in SEQ ID NO.15, or an amino acid sequence having at least 75% identity to the amino acid sequence; and, the amino acid sequence as shown in SEQ ID NO.36, or an amino acid sequence having at least 75% identity to the amino acid sequence;

(1-10) the amino acid sequence as shown in SEQ ID NO.15, or an amino acid sequence having at least 75% identity to the amino acid sequence; and, the amino acid sequence as shown in SEQ ID NO.37, or an amino acid sequence having at least 75% identity to the amino acid sequence;

(1-11) the amino acid sequence as shown in SEQ ID NO.15, or an amino acid sequence having at least 75% identity to the amino acid sequence; and, the amino acid sequence as shown in SEQ ID NO.38, or an amino acid sequence having at least 75% identity to the amino acid sequence;

(1-12) the amino acid sequence as shown in SEQ ID NO.15, or an amino acid sequence having at least 75% identity to the amino acid sequence; and, the amino acid sequence as shown in SEQ ID NO.39, or an amino acid sequence having at least 75% identity to the amino acid sequence;

(1-13) the amino acid sequence as shown in SEQ ID NO.15, or an amino acid sequence having at least 75% identity to the amino acid sequence; and, the amino acid sequence as shown in SEQ ID NO.40, or an amino acid sequence having at least 75% identity to the amino acid sequence;

(1-14) the amino acid sequence as shown in SEQ ID NO.15, or an amino acid sequence having at least 75% identity to the amino acid sequence; and, the amino acid sequence as shown in SEQ ID NO.41, or an amino acid sequence having at least 75% identity to the amino acid sequence;

(1-15) the amino acid sequence as shown in SEQ ID NO.15, or an amino acid sequence having at least 75% identity to the amino acid sequence; and, the amino acid sequence as shown in SEQ ID NO.42, or an amino acid sequence having at least 75% identity to the amino acid sequence;

(1-16) the amino acid sequence as shown in SEQ ID NO.19, or an amino acid sequence having at least 75% identity to the amino acid sequence; and, the amino acid sequence as shown in SEQ ID NO.40, or an amino acid sequence having at least 75% identity to the amino acid sequence;

(1-17) the amino acid sequence as shown in SEQ ID NO.20, or an amino acid sequence having at least 75% identity to the amino acid sequence; and, the amino acid sequence as shown in SEQ ID NO.40, or an amino acid sequence having at least 75% identity to the amino acid sequence;

(1-18) the amino acid sequence as shown in SEQ ID NO.21, or an amino acid sequence having at least 75% identity to the amino acid sequence; and, the amino acid sequence as shown in SEQ ID NO.40, or an amino acid sequence having at least 75% identity to the amino acid sequence;

(1-19) the amino acid sequence as shown in SEQ ID NO.22, or an amino acid sequence having at least 75% identity to the amino acid sequence; and, the amino acid sequence as shown in SEQ ID NO.40, or an amino acid sequence having at least 75% identity to the amino acid sequence;

(1-20) the amino acid sequence as shown in SEQ ID NO.23, or an amino acid sequence having at least 75% identity to the amino acid sequence; and, the amino acid sequence as shown in SEQ ID NO.40, or an amino acid sequence having at least 75% identity to the amino acid sequence;

(1-21) the amino acid sequence as shown in SEQ ID NO.15, or an amino acid sequence having at least 75% identity to the amino acid sequence; and, the amino acid sequence as shown in SEQ ID NO.43, or an amino acid sequence having at least 75% identity to the amino acid sequence;

(1-22) the amino acid sequence as shown in SEQ ID NO.15, or an amino acid sequence having at least 75% identity to the amino acid sequence; and, the amino acid sequence as shown in SEQ ID NO.44, or an amino acid sequence having at least 75% identity to the amino acid sequence;

(1-23) the amino acid sequence as shown in SEQ ID NO.15, or an amino acid sequence having at least 75% identity to the amino acid sequence; and, the amino acid sequence as shown in SEQ ID NO.45, or an amino acid sequence having at least 75% identity to the amino acid sequence;

(1-24) the amino acid sequence as shown in SEQ ID NO.15, or an amino acid sequence having at least 75% identity to the amino acid sequence; and, the amino acid sequence as shown in SEQ ID NO.46, or an amino acid sequence having at least 75% identity to the amino acid sequence;

(1-25) the amino acid sequence as shown in SEQ ID NO.23, or an amino acid sequence having at least 75% identity to the amino acid sequence; and, the amino acid sequence as shown in SEQ ID NO.43, or an amino acid sequence having at least 75% identity to the amino acid sequence;

(1-26) the amino acid sequence as shown in SEQ ID NO.23, or an amino acid sequence having at least 75% identity to the amino acid sequence; and, the amino acid sequence as shown in SEQ ID NO.44, or an amino acid sequence having at least 75% identity to the amino acid sequence;

(1-27) the amino acid sequence as shown in SEQ ID NO.23, or an amino acid sequence having at least 75% identity to the amino acid sequence; and, the amino acid sequence as shown in SEQ ID NO.45, or an amino acid sequence having at least 75% identity to the amino acid sequence;

(1-28) the amino acid sequence as shown in SEQ ID NO.23, or an amino acid sequence having at least 75% identity to the amino acid sequence; and, the amino acid sequence as shown in SEQ ID NO.46, or an amino acid sequence having at least 75% identity to the amino acid sequence;

(1-29) the amino acid sequence as shown in SEQ ID NO.24, or an amino acid sequence having at least 75% identity to the amino acid sequence; and, the amino acid sequence as shown in SEQ ID NO.44, or an amino acid sequence having at least 75% identity to the amino acid sequence;

(1-30) the amino acid sequence as shown in SEQ ID NO.25, or an amino acid sequence having at least 75% identity to the amino acid sequence; and, the amino acid sequence as shown in SEQ ID NO.44, or an amino acid sequence having at least 75% identity to the amino acid sequence;

(1-31) the amino acid sequence as shown in SEQ ID NO.26, or an amino acid sequence having at least 75% identity to the amino acid sequence; and, the amino acid sequence as shown in SEQ ID NO.44, or an amino acid sequence having at least 75% identity to the amino acid sequence;

(1-32) the amino acid sequence as shown in SEQ ID NO.27, or an amino acid sequence having at least 75% identity to the amino acid sequence; and, the amino acid sequence as shown in SEQ ID NO.44, or an amino acid sequence having at least 75% identity to the amino acid sequence;

(1-33) the amino acid sequence as shown in SEQ ID NO.28, or an amino acid sequence having at least 75% identity to the amino acid sequence; and, the amino acid sequence as shown in SEQ ID NO.44, or an amino acid sequence having at least 75% identity to the amino acid sequence;

(1-34) the amino acid sequence as shown in SEQ ID NO.29, or an amino acid sequence having at least 75% identity to the amino acid sequence; and, the amino acid sequence as shown in SEQ ID NO.44, or an amino acid sequence having at least 75% identity to the amino acid sequence;

(1-35) the amino acid sequence as shown in SEQ ID NO.30, or an amino acid sequence having at least 75% identity to the amino acid sequence; and, the amino acid sequence as shown in SEQ ID NO.44, or an amino acid sequence having at least 75% identity to the amino acid sequence;

(1-36) the amino acid sequence as shown in SEQ ID NO.31, or an amino acid sequence having at least 75% identity to the amino acid sequence; and, the amino acid sequence as shown in SEQ ID NO.44, or an amino acid sequence having at least 75% identity to the amino acid sequence;

(1-37) the amino acid sequence as shown in SEQ ID NO.32, or an amino acid sequence having at least 75% identity to the amino acid sequence; and, the amino acid sequence as shown in SEQ ID NO.44, or an amino acid sequence having at least 75% identity to the amino acid sequence; and

(1-38) the amino acid sequence as shown in SEQ ID NO.33, or an amino acid sequence having at least 75% identity to the amino acid sequence; and, the amino acid sequence as shown in SEQ ID NO.44, or an amino acid sequence having at least 75% identity to the amino acid sequence;

alternatively,

(2)

(2-1) the amino acid sequence as shown in SEQ ID NO.5, or an amino acid sequence having at least 75% identity to the amino acid sequence; and, the amino acid sequence as shown in SEQ ID NO.6, or an amino acid sequence having at least 75% identity to the amino acid sequence;

(2-2) the amino acid sequence as shown in SEQ ID NO.9, or an amino acid sequence having at least 75% identity to the amino acid sequence; and, the amino acid sequence as shown in SEQ ID NO.10, or an amino acid sequence having at least 75% identity to the amino acid sequence;

(2-3) the amino acid sequence as shown in SEQ ID NO.9, or an amino acid sequence having at least 75% identity to the amino acid sequence; and, the amino acid sequence as shown in SEQ ID NO.69, or an amino acid sequence having at least 75% identity to the amino acid sequence;

(2-4) the amino acid sequence as shown in SEQ ID NO.9, or an amino acid sequence having at least 75% identity to the amino acid sequence; and, the amino acid sequence as shown in SEQ ID NO.70, or an amino acid sequence having at least 75% identity to the amino acid sequence;

(2-5) the amino acid sequence as shown in SEQ ID NO.9, or an amino acid sequence having at least 75% identity to the amino acid sequence; and, the amino acid sequence as shown in SEQ ID NO.71, or an amino acid sequence having at least 75% identity to the amino acid sequence;

(2-6) the amino acid sequence as shown in SEQ ID NO.9, or an amino acid sequence having at least 75% identity to the amino acid sequence; and, the amino acid sequence as shown in SEQ ID NO.72, or an amino acid sequence having at least 75% identity to the amino acid sequence;

(2-7) the amino acid sequence as shown in SEQ ID NO.9, or an amino acid sequence having at least 75% identity to the amino acid sequence; and, the amino acid sequence as shown in SEQ ID NO.73, or an amino acid sequence having at least 75% identity to the amino acid sequence;

(2-8) the amino acid sequence as shown in SEQ ID NO.9, or an amino acid sequence having at least 75% identity to the amino acid sequence; and, the amino acid sequence as shown in SEQ ID NO.74, or an amino acid sequence having at least 75% identity to the amino acid sequence;

(2-9) the amino acid sequence as shown in SEQ ID NO.9, or an amino acid sequence having at least 75% identity to the amino acid sequence; and, the amino acid sequence as shown in SEQ ID NO.76, or an amino acid sequence having at least 75% identity to the amino acid sequence;

(2-10) the amino acid sequence as shown in SEQ ID NO.9, or an amino acid sequence having at least 75% identity to the amino acid sequence; and, the amino acid sequence as shown in SEQ ID NO.77, or an amino acid sequence having at least 75% identity to the amino acid sequence;

(2-11) the amino acid sequence as shown in SEQ ID NO.9, or an amino acid sequence having at least 75% identity to the amino acid sequence; and, the amino acid sequence as shown in SEQ ID NO.78, or an amino acid sequence having at least 75% identity to the amino acid sequence;

(2-12) the amino acid sequence as shown in SEQ ID NO.9, or an amino acid sequence having at least 75% identity to the amino acid sequence; and, the amino acid sequence as shown in SEQ ID NO.79, or an amino acid sequence having at least 75% identity to the amino acid sequence;

(2-13) the amino acid sequence as shown in SEQ ID NO.9, or an amino acid sequence having at least 75% identity to the amino acid sequence; and, the amino acid sequence as shown in SEQ ID NO.80, or an amino acid sequence having at least 75% identity to the amino acid sequence;

(2-14) the amino acid sequence as shown in SEQ ID NO.9, or an amino acid sequence having at least 75% identity to the amino acid sequence; and, the amino acid sequence as shown in SEQ ID NO.81, or an amino acid sequence having at least 75% identity to the amino acid sequence;

(2-15) the amino acid sequence as shown in SEQ ID NO.49, or an amino acid sequence having at least 75% identity to the amino acid sequence; and, the amino acid sequence as shown in SEQ ID NO.10, or an amino acid sequence having at least 75% identity to the amino acid sequence;

(2-16) the amino acid sequence as shown in SEQ ID NO.50, or an amino acid sequence having at least 75% identity to the amino acid sequence; and, the amino acid sequence as shown in SEQ ID NO.10, or an amino acid sequence having at least 75% identity to the amino acid sequence;

(2-17) the amino acid sequence as shown in SEQ ID NO.49, or an amino acid sequence having at least 75% identity to the amino acid sequence; and, the amino acid sequence as shown in SEQ ID NO.75, or an amino acid sequence having at least 75% identity to the amino acid sequence;

(2-18) the amino acid sequence as shown in SEQ ID NO.49, or an amino acid sequence having at least 75% identity to the amino acid sequence; and, the amino acid sequence as shown in SEQ ID NO.82, or an amino acid sequence having at least 75% identity to the amino acid sequence;

(2-19) the amino acid sequence as shown in SEQ ID NO.49, or an amino acid sequence having at least 75% identity to the amino acid sequence; and, the amino acid sequence as shown in SEQ ID NO.83, or an amino acid sequence having at least 75% identity to the amino acid sequence;

(2-20) the amino acid sequence as shown in SEQ ID NO.49, or an amino acid sequence having at least 75% identity to the amino acid sequence; and, the amino acid sequence as shown in SEQ ID NO.77, or an amino acid sequence having at least 75% identity to the amino acid sequence;

(2-21) the amino acid sequence as shown in SEQ ID NO.49, or an amino acid sequence having at least 75% identity to the amino acid sequence; and, the amino acid sequence as shown in SEQ ID NO.79, or an amino acid sequence having at least 75% identity to the amino acid sequence;

(2-22) the amino acid sequence as shown in SEQ ID NO.50, or an amino acid sequence having at least 75% identity to the amino acid sequence; and, the amino acid sequence as shown in SEQ ID NO.77, or an amino acid sequence having at least 75% identity to the amino acid sequence;

(2-23) the amino acid sequence as shown in SEQ ID NO.50, or an amino acid sequence having at least 75% identity to the amino acid sequence; and, the amino acid sequence as shown in SEQ ID NO.79, or an amino acid sequence having at least 75% identity to the amino acid sequence;

(2-24) the amino acid sequence as shown in SEQ ID NO.53, or an amino acid sequence having at least 75% identity to the amino acid sequence; and, the amino acid sequence as shown in SEQ ID NO.77, or an amino acid sequence having at least 75% identity to the amino acid sequence;

(2-25) the amino acid sequence as shown in SEQ ID NO.9, or an amino acid sequence having at least 75% identity to the amino acid sequence; and, the amino acid sequence as shown in SEQ ID NO.84, or an amino acid sequence having at least 75% identity to the amino acid sequence;

(2-26) the amino acid sequence as shown in SEQ ID NO.9, or an amino acid sequence having at least 75% identity to the amino acid sequence; and, the amino acid sequence as shown in SEQ ID NO.85, or an amino acid sequence having at least 75% identity to the amino acid sequence;

(2-27) the amino acid sequence as shown in SEQ ID NO.51, or an amino acid sequence having at least 75% identity to the amino acid sequence; and, the amino acid sequence as shown in SEQ ID NO.77, or an amino acid sequence having at least 75% identity to the amino acid sequence;

(2-28) the amino acid sequence as shown in SEQ ID NO.52, or an amino acid sequence having at least 75% identity to the amino acid sequence; and, the amino acid sequence as shown in SEQ ID NO.77, or an amino acid sequence having at least 75% identity to the amino acid sequence;

(2-29) the amino acid sequence as shown in SEQ ID NO.54, or an amino acid sequence having at least 75% identity to the amino acid sequence; and, the amino acid sequence as shown in SEQ ID NO.77, or an amino acid sequence having at least 75% identity to the amino acid sequence;

(2-30) the amino acid sequence as shown in SEQ ID NO.9, or an amino acid sequence having at least 75% identity to the amino acid sequence; and, the amino acid sequence as shown in SEQ ID NO.86, or an amino acid sequence having at least 75% identity to the amino acid sequence;

(2-31) the amino acid sequence as shown in SEQ ID NO.52, or an amino acid sequence having at least 75% identity to the amino acid sequence; and, the amino acid sequence as shown in SEQ ID NO.86, or an amino acid sequence having at least 75% identity to the amino acid sequence;

(2-32) the amino acid sequence as shown in SEQ ID NO.55, or an amino acid sequence having at least 75% identity to the amino acid sequence; and, the amino acid sequence as shown in SEQ ID NO.87, or an amino acid sequence having at least 75% identity to the amino acid sequence;

(2-33) the amino acid sequence as shown in SEQ ID NO.56, or an amino acid sequence having at least 75% identity to the amino acid sequence; and, the amino acid sequence as shown in SEQ ID NO.87, or an amino acid sequence having at least 75% identity to the amino acid sequence;

(2-34) the amino acid sequence as shown in SEQ ID NO.57, or an amino acid sequence having at least 75% identity to the amino acid sequence; and, the amino acid sequence as shown in SEQ ID NO.87, or an amino acid sequence having at least 75% identity to the amino acid sequence;

(2-35) the amino acid sequence as shown in SEQ ID NO.51, or an amino acid sequence having at least 75% identity to the amino acid sequence; and, the amino acid sequence as shown in SEQ ID NO.87, or an amino acid sequence having at least 75% identity to the amino acid sequence;

(2-36) the amino acid sequence as shown in SEQ ID NO.54, or an amino acid sequence having at least 75% identity to the amino acid sequence; and, the amino acid sequence as shown in SEQ ID NO.87, or an amino acid sequence having at least 75% identity to the amino acid sequence;

(2-37) the amino acid sequence as shown in SEQ ID NO.58, or an amino acid sequence having at least 75% identity to the amino acid sequence; and, the amino acid sequence as shown in SEQ ID NO.87, or an amino acid sequence having at least 75% identity to the amino acid sequence;

(2-38) the amino acid sequence as shown in SEQ ID NO.59, or an amino acid sequence having at least 75% identity to the amino acid sequence; and, the amino acid sequence as shown in SEQ ID NO.87, or an amino acid sequence having at least 75% identity to the amino acid sequence;

(2-39) the amino acid sequence as shown in SEQ ID NO.60, or an amino acid sequence having at least 75% identity to the amino acid sequence; and, the amino acid sequence as shown in SEQ ID NO.87, or an amino acid sequence having at least 75% identity to the amino acid sequence;

(2-40) the amino acid sequence as shown in SEQ ID NO.61, or an amino acid sequence having at least 75% identity to the amino acid sequence; and, the amino acid sequence as shown in SEQ ID NO.87, or an amino acid sequence having at least 75% identity to the amino acid sequence;

(2-41) the amino acid sequence as shown in SEQ ID NO.62, or an amino acid sequence having at least 75% identity to the amino acid sequence; and, the amino acid sequence as shown in SEQ ID NO.87, or an amino acid sequence having at least 75% identity to the amino acid sequence;

(2-42) the amino acid sequence as shown in SEQ ID NO.63, or an amino acid sequence having at least 75% identity to the amino acid sequence; and, the amino acid sequence as shown in SEQ ID NO.87, or an amino acid sequence having at least 75% identity to the amino acid sequence;

(2-43) the amino acid sequence as shown in SEQ ID NO.64, or an amino acid sequence having at least 75% identity to the amino acid sequence; and, the amino acid sequence as shown in SEQ ID NO.87, or an amino acid sequence having at least 75% identity to the amino acid sequence;

(2-44) the amino acid sequence as shown in SEQ ID NO.65, or an amino acid sequence having at least 75% identity to the amino acid sequence; and, the amino acid sequence as shown in SEQ ID NO.87, or an amino acid sequence having at least 75% identity to the amino acid sequence;

(2-45) the amino acid sequence as shown in SEQ ID NO.66, or an amino acid sequence having at least 75% identity to the amino acid sequence; and, the amino acid sequence as shown in SEQ ID NO.87, or an amino acid sequence having at least 75% identity to the amino acid sequence;

(2-46) the amino acid sequence as shown in SEQ ID NO.67, or an amino acid sequence having at least 75% identity to the amino acid sequence; and, the amino acid sequence as shown in SEQ ID NO.87, or an amino acid sequence having at least 75% identity to the amino acid sequence; and

(2-47) the amino acid sequence as shown in SEQ ID NO.68, or an amino acid sequence having at least 75% identity to the amino acid sequence; and, the amino acid sequence as shown in SEQ ID NO.87, or an amino acid sequence having at least 75% identity to the amino acid sequence.

With respect to the antigen, the antibody or fragment thereof provided by the present disclosure is an anti-IL-11 antibody or antigen binding fragment thereof. Preferably, the antibody is a murine antibody, a rabbit antibody, a human antibody, a chimeric antibody or a fully or partially humanized antibody. The antibody may also be a derivatized antibody, e.g., antibodies obtained through CDRs grafting, affinity maturation, point mutation engineering, chemical modification, and the like conducted on the original murine monoclonal antibodies, in which the chemical modification includes glycosylation, acetylation, pegylation, phosphorylation, amidation, cleavage by protease, attachment to a cellular ligand or effector molecule, protection and/or blocking of an active reactive group, and the like. Preferably, the antigen binding fragment of an anti-IL-11 antibody may be a fragment of the antibody in any form, e.g., single-chain variable fragment (scFv), bivalent single-chain variable fragment (BsFv), disulfide-stabilized variable fragment (dsFv), (disulfide-stabilized variable fragment)2 ((dsFv)$_2$), antigen-binding fragment (Fab), Fab' fragment (Fab'), F(ab')$_2$ fragment (F(ab')$_2$), or variable fragment (Fv).

In addition to the variable regions, the antibody or fragment thereof provided by the present disclosure further comprises a heavy chain constant region (CH) and/or a light chain constant region (CL), preferably a human or murine heavy chain constant region and/or light chain constant region. Preferably, the antibody or fragment thereof comprises a heavy chain constant region of an IgG, IgA, IgM, IgD or IgE and/or a kappa or lambda type light chain constant region.

According to particular embodiments of the present disclosure, the antibody is a monoclonal antibody, preferably a murine, chimeric or humanized monoclonal antibody. According to particular embodiments of the present disclosure, the monoclonal antibody comprises the sequence of murine IgG1 heavy chain constant region, e.g., that as shown in SEQ ID NO.3; and/or comprises the sequence of murine light chain constant region, e.g., that as shown in SEQ ID NO.4. According to particular embodiments of the present disclosure, the monoclonal antibody comprises a human heavy chain constant region and a human light chain constant region, e.g., those as shown in SEQ ID NO. 13 and SEQ ID NO. 14, respectively.

According to particular embodiments of the present disclosure, the anti-IL-11 antibody provided by the present disclosure is a monoclonal antibody. Preferably, the anti-IL-11 antibody provided by the present disclosure is an immunoglobulin. For example, the immunoglobulin is a human type of IgA, IgD, IgE, IgG or IgM, more preferably a human IgG1, IgG2, IgG3 or IgG4 subtype.

In another aspect, the present disclosure provides a nucleic acid molecule comprising a nucleotide sequence encoding the heavy chain CDRs, the light chain CDRs, the heavy chain variable region, the light chain variable region, the heavy chain or the light chain comprised in the antibody or fragment thereof provided by the present disclosure.

The nucleic acid molecule provided by the present disclosure can be cloned into a vector which in turn can be used to transfect or transform a host cell. Accordingly, in yet another aspect, the present disclosure provides a vector comprising the nucleic acid molecule provided by the present disclosure. The vector can be a eukaryotic expression vector, a prokaryotic expression vector, an artificial chromosome, a phage vector and the like. The vector or nucleic acid molecule provided by the present disclosure can be used to transform or transfect a host cell, for antibody preservation or expression, etc. Thus, in a further aspect, the present disclosure provides a host cell comprising the nucleic acid molecule and/or the vector provided by the present disclosure, or transformed or transfected with the nucleic acid molecule and/or the vector provided by the present disclosure. The host cell may be any prokaryotic or eukaryotic cell, such as a bacterial or insect, fungus, plant or animal cell.

The antibody or fragment thereof provided by the present disclosure can be obtained using any conventional techniques known in the art. For example, the host cell provided by the present disclosure is cultured under conditions that allow the host cell to express the heavy chain and the light chain of the antibody. Optionally, the method further includes a step of recovering the produced antibody.

The antibody or fragment thereof, the nucleic acid molecule, the vector, and/or the host cell provided by the present disclosure can be contained in a composition, more particularly in a pharmaceutical composition, e.g., a pharmaceutical preparation, to be used for various purposes as actually needed.

Thus, in still a further aspect, the present disclosure also provides a composition comprising the antibody or fragment thereof, the nucleic acid molecule, the vector, and/or the host cell provided by the present disclosure, optionally a pharmaceutically acceptable accessory material. The composition provided by the present disclosure may be formulated into various dosage forms known in the medical or pharmaceutical arts and administered via a suitable manner.

In yet another aspect, the present disclosure also provides use of the antibody or fragment thereof, the nucleic acid molecule, the vector, the host cell and/or the composition in the manufacture of a medicament for preventing, treating, and/or ameliorating a disease or disorder associated with the expression (including overexpression) of IL-11. The antibody or fragment thereof, the nucleic acid molecule, the vector, the host cell and/or the composition may function through binding to IL-11 and blocking the formation of an IL-1I/IL-11Rα/GP130 complex and inhibiting the activation of a STAT3 signaling pathway, among the others. According to particular embodiments of the present disclosure, the disease or disorder is an inflammatory disease or a tumor, e.g., liver injury, pulmonary fibrosis and colon cancer.

Accordingly, the present disclosure also provides a method for preventing, treating and/or ameliorating a disease or disorder associated with the expression (including overexpression) of IL-11, comprising administering to a subject in need thereof the antibody or fragment thereof, the nucleic acid molecule, the vector, the host cell and/or the composition provided by the present disclosure. Preferably, the disease or disorder is an inflammatory disease or a tumor, e.g., liver injury, pulmonary fibrosis and colon cancer. Preferably, the subject is a mammal; more preferably, the subject is a human.

The method for preventing, treating and/or ameliorating a disease or disorder provided by the present disclosure will be used depending on various factors, including the specific active ingredient of the pharmaceutical composition to be administered, the age, body weight, sex or physical and medical condition of the patient, the severity of the condition to be treated, the administration route, and the like.

In still a further aspect, the present disclosure also provides use of the antibody or fragment thereof, the nucleic acid molecule, the vector, the host cell and/or the composition provided by the present disclosure in the manufacture of an agent for diagnosing a disease or disorder associated with the expression (including overexpression) of IL-11. Preferably, the disease or disorder is an inflammatory disease or a tumor, e.g., liver injury, pulmonary fibrosis and colon cancer.

In yet another aspect, the present disclosure provides a kit comprising the antibody or fragment thereof, the nucleic acid molecule, the vector, the host cell and/or the composition provided by the present disclosure. The kit may be used for the treating or diagnosing as above, or used for detecting the antigen. For example, the kit is a kit for detecting the expression of IL-11 in any biological sample using ELISA.

## BRIEF DESCRIPTION OF THE DRAWINGS

Embodiments of the invention are described in detail below with reference to the attached figures, in which:

FIG. 1 shows the binding activity of hybridoma supernatants to human and murine IL-11.

FIG. 2 shows the blocking activity of anti-IL-11 antibodies on the formation of IL-11/IL-11R$\alpha$/GP130, in which 2a: mu8C8; 2b: mu18A10; 2c: 3C6-mFc.

FIG. 3 shows the analysis results of the expression of reporter gene in STAT3/IL-11R$\alpha$/GP130-HEK293 cells stimulated by IL-11.

FIG. 4 shows the in vivo activity of anti-IL-11 antibodies in a mouse model of bleomycin (BLM)-induced pulmonary fibrosis, in which 4a: H&E and Masson staining results of lung tissue sections; 4b: analysis results of fibrosis and lung function.

FIG. 5 shows the efficacy of anti-IL-11 antibodies in a tumor model of mouse bearing MC38 tumor cells, in which 5a: tumor volume; 5b: tumor weight.

## DETAILED DESCRIPTION OF PREFERRED EMBODIMENTS

The invention is illustrated below with reference to specific examples. It will be understood by those skilled in the art that these examples are merely illustrative of the present invention and do not limit the scope of the present invention in any way.

The experimental procedures in the following examples are all conventional ones unless otherwise specified. The raw materials, reagent materials and the like used in the following examples are all commercially available products unless otherwise specified.

### Example 1 Preparation and analysis of hybridoma-derived murine anti-IL-11 antibodies

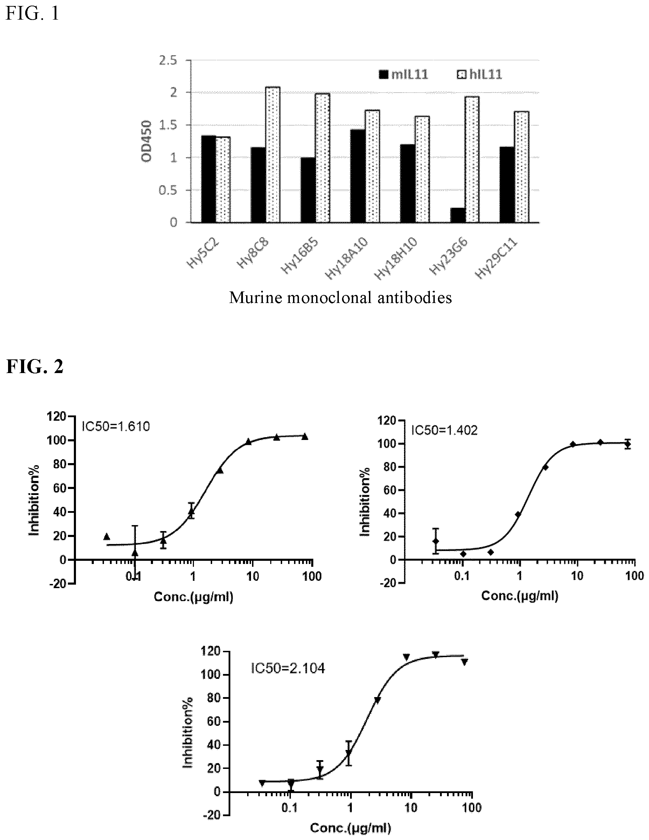

Murine anti-IL-11 hybridoma monoclones 5C2, 8C8, 16B5, 18A10, 18H10, 23G6 and 29C11 were obtained by murine hybridoma technology. The binding activity of the supernatants of those clones to human IL-11 (AAH12506.1, Pro22-Leu199; SEQ ID NO.128) and murine IL-11 (NP_032376.1, Pro22-Leu199; SEQ ID NO.129) immobilized on the surface of ELISA plates was detected via direct ELISA. The results are shown in FIG. 1.

When the hybridoma cells were cultured to a certain number, culture supernatants were collected and purified using Protein G affinity chromatography to obtain murine hybridoma antibodies (Hy5C2, Hy8C8, Hy16B5, Hy18A10, Hy18H10, Hy23G6 and Hy29C11). The affinity of the murine antibodies purified from the hybridoma supernatants was determined using an Octet QKe System instrument from Fortebio Inc. with an anti-mouse IgG Fc Capture biosensor to capture the Fc fragments of the antibodies, for which an anti-IL-11 antibody named "3C6-mFc" recombinantly expressed was used at the same concentration as a positive control. Specifically, a hybridoma supernatant was diluted with HBS-EP+ buffer (GE), and was allowed to pass through the surface of AMC probes (Cat: 18-5088, PALL), and so the antibody in the hybridoma supernatant was captured on the surface of the probes; then, human IL-11 diluted in the HBS-EP+ buffer (300 nM) was used as a mobile phase to react with the antibody captured on the surface of the probes. Both the binding and the dissociating were conducted for 300 seconds. When the experiment was finished, data from which response values of blank controls had been deducted was fitted using a 1: 1 Langmuir binding model by software, and then kinetic constants for antigen-antibody binding were calculated. Binding signals were detected on the Octet QKe System instrument with Hy5C2, Hy8C8, Hy16B5, Hy18A10, and Hy18H10. Final results of the measurement are shown in Table 1 below.

The sequences of the positive control anti-IL-11 antibody 3C6-mFc are disclosed in the patent publication NO. WO2019/238882A1. The sequence of the heavy chain variable region of 3C6-mFc is named as "3C6-VH" and as shown in SEQ ID NO. 1, and the sequence of the light chain variable region of 3C6-mFc is named as "3C6-VL" and as shown in SEQ ID NO.2; and 3C6-mFc has murine IgG1 constant regions, i.e., the heavy chain constant region of murine antibody as shown in SEQ ID NO.3 and the light chain constant region of murine antibody as shown in SEQ ID NO. 4.

Table 1. Results of affinity of the hybridoma supernatants detected on Fortebio

| Sample | KD (M) | kon (1/Ms) | kdis (1/s) |
|---|---|---|---|
| 3C6-mFc (positive control) | 7.12E-10 | 8.16E+05 | 5.81E-04 |
| Hy5C2 | 1.61E-08 | 1.30E+06 | 2.09E-02 |
| Hy8C8 | 1.30E-09 | 1.51E+06 | 2.04E-03 |
| Hy 16B5 | 2.00E-08 | 4.48E+05 | 8.95E-03 |
| Hy18A10 | 1.09E-09 | 1.71E+06 | 1.87E-03 |
| Hy18H10 | 1.65E-07 | 1.37E+05 | 2.26E-02 |

SEQ ID NO.1 3C6-VH

EVQLVQSGAEVKKPGASVKISCKASGYTFTDYNMDWVKQAPGQRLEWIGDINPHNG

GPIYNQKFTGRATLTVDKSASTAYMELSSLRSEDTAVYYCARGELGHWYFDVWGQGT

TVTVSS

SEQ ID NO.2 3C6-VL

DIVLTQSPASLALSPGERATLSCRASKSVSTSGYSYIHWYQQKPGQAPRLLIYLASNLDS

GVPARFSGSGSGTDFTLTISSLEEEDFATYYCQHSRDLPPTFGQGTKLEIK

SEQ ID NO.3 heavy chain constant region of murine antibody

AKTTPPSVYPLAPGSAAQTNSMVTLGCLVKGYFPEPVTVTWNSGSLSSGVHTFPAVLQ

SDLYTLSSSVTVPSSTWPSETVTCNVAHPASSTKVDKKIVPRDCGCKPCICTVPEVSSVFI

FPPKPKDVLTITLTPKVTCVVVDISKDDPEVQFSWFVDDVEVHTAQTQPREEQFNSTFR

SVSELPIMHQDWLNGKEFKCRVNSAAFPAPIEKTISKTKGRPKAPQVYTIPPPKEQMAK

DKVSLTCMITDFFPEDITVEWQWNGQPAENYKNTQPIMDTDGSYFVYSKLNVQKSNW

EAGNTFTCSVLHEGLHNHHTEKSLSHSPGK

SEQ ID NO.4 light chain constant region of murine antibody

RTVAAPTVSIFPPSSEQLTSGGASVVCFLNNFYPKDINVKWKIDGSERQNGVLNSWTDQ

DSKDSTYSMSSTLTLTKDEYERHNSYTCEATHKTSTSPIVKSFNRNEC

**Example 2 Gene cloning and recombinant expression of murine anti-IL 11 antibodies**

When the hybridoma cells were cultured to a certain number, the cells were collected, and total RNA was extracted from the cells. The RNA obtained was subjected to reverse transcription PCR, and genes encoding the light and heavy chain variable regions of the murine antibodies were obtained and sequenced. The sequence of the heavy chain variable region of Hy18A10 is named as "mu18A10 VH" and as shown in SEQ ID NO.5, and the sequence of the light chain variable region of Hy18A10 is named as "mu18A10 VL" and as shown in SEQ ID NO.6. The sequence of the heavy chain variable region of Hy8C8 is named as "mu8C8 VH" and as shown in SEQ ID NO.7, and the sequence of the light chain variable region of Hy8C8 is named as "mu8C8 VL" and as shown in SEQ ID NO.8. CDRs were defined according to Kabat numbering and are shown underlined; the same is true hereinafter.

SEQ ID NO.5 (CDRs: SEQ ID NO.88/89/90) mu18A10-VH

QVQLQQPGAELVKPGASVKMSCKASGYKFI<u>TYNMD</u>WVKQTPGQGLEWIG<u>TIYPGNG DTSYNQKFKG</u>KATLTADKSSSTAYMQLSSLTSEDSAVYYCAR<u>GDLYALDY</u>WGQGTSVT VSS

SEQ ID NO.6 (CDRs: SEQ ID NO.91/92/93) mu18A10-VL

SIVMTQTPKFLLVSAGDRVTITC<u>KASQSVSNDVA</u>WYQQKPGQSPKLLIY<u>SASNRYT</u>GVP DRFTGSGYGTDFTFTINTVQAEDLAVYFC<u>QHDYSSPYT</u>FGGGTKLEIKR

SEQ ID NO.7 (CDRs: SEQ ID NO.94/100/96) mu8C8-VH

DVQLVESGGGLVQPGGSRKLSCAASGFTFS<u>SFGMH</u>WVRQSPERGLEWVA<u>YITSGSNTI YYADTVKG</u>RFTISRDNPENTLFLQMTSLRSEDTAMYYCAR<u>EYYSYDEGFAY</u>WGQGTL VTVSA

SEQ ID NO.8 (CDRs: SEQ ID NO.97/98/99) mu8C8-VL

EIVLTQSPTTMAASPGEKITITC<u>SVSSSISSNYLH</u>WYQQKPGFSPKLLIY<u>RTSNLAS</u>GVPA RFSGSGSGTSHSLTIDTMEAEDVATYYC<u>QQGVDVPFT</u>FGSGTKLEIKR

According to the amino acid sequences of the murine antibodies obtained from the gene cloning, antibody coding genes were optimized and synthesized. Cleavage sites of restriction enzymes were designed and introduced into both ends of the genes, and the genes encoding the variable regions of the antibodies were linked with the genes encoding the heavy and light chain constant regions of murine antibody (the amino acid sequences are as shown in SEQ ID NO.3 and SEQ ID NO.4) through enzyme digestion. The obtained genes were cloned into a eukaryotic cell transient expression vector respectively, and the expression vectors of recombinant murine antibodies were obtained. The light chain and heavy chain expression vectors of the antibodies were then transformed into E. coli for plasmid proliferation and a large number of plasmids were isolated. The plasmids of the heavy and light chains of the candidate antibodies were co-transfected into HEK293 cells according to the instructions of 293fectin Transfection Reagent, to recombinantly express the antibodies. 5-6 days after the transfection of the cells, culture supernatants were collected and purified with Protein G affinity chromatography column, and recombinant murine antibodies mu8C8 and mu18A10 were obtained.

**Example 3 Analysis of the activity of recombinant murine anti-IL-11 antibodies for blocking the formation of IL-11/IL-11Rα/GP130 complex**

The activity of the recombinant murine anti-IL-11 antibodies for blocking the formation of IL-11/IL-11Rα/GP130 complex was analyzed via competition ELISA. IL-11Rα-His (NP_004503.1, Met1-Val363, C-terminus-6×His Tag) was firstly added into ELISA plates to be coated overnight. Next day, samples were prepared as follows: IL-11 (Cat: 12225-HNCE, Sino Biological Inc.) at a constant concentration of 0.4μg/ml was firstly mixed and co-incubated with a murine anti-IL-11 antibody gradiently diluted (working concentrations: 75, 25, 8.33, 2.78, 0.926, 0.309, and 0.103 μg/ml) and then mixed with gp130-hFc (NP_034690.3, Met1-Glu617, C-terminus-mFc) at a final concentration of 1 μg/ml, and then the mixtures obtained were added into the ELISA plates. After co-incubating and washing, an HRP conjugated anti-human Fc secondary antibody was added to develop the color which was then detected. Finally, the results showed that the murine antibodies mu8C8 and mu18A10 had a rather good blocking effect, as indicated by their IC50 values of 1.610 and 1.402

μg/ml respectively (2a and 2b in FIG. 2), comparable to that of the control antibody 3C6-mFc (2.104 μg/ml, 2c in FIG. 2).

**Example 4 Humanization of antibodies**

The murine antibodies mu8C8 and mu18A10 were engineered for humanization and humanized antibodies were recombinantly expressed.

Heavy and light chain CDRs of the murine antibodies mu8C8 and mu18A10 were grafted into a human template having a high homology with the murine antibodies, and back mutation was performed if necessary, and then humanized antibodies named as "hz18A10" and "hz8C8" were obtained. The sequence of the heavy chain variable region of hz18A10 is named as "hz18A10-VH" and as shown in SEQ ID NO.9, and the sequence of the light chain variable region of hz 18A 10 is named as "hz18A10-VL" and as shown in SEQ ID NO.10. The sequence of the heavy chain variable region of "hz8C8" is named as "hz8C8-VH" and as shown in SEQ ID NO.11, and the sequence of the light chain variable region of "hz8C8" is named as "hz8C8-VL" and as shown in SEQ ID NO.12.

SEQ ID NO.9 (CDRs: SEQ ID NO.88/89/90): hz18A10-VH

EVQLVQSGAEVKKPGASVKVSCKASGYTFT<u>TYNMD</u>WVRQAPGQGLEWMG<u>TIYPGN GDTSYNQKFKG</u>RVTMTRDTSTSTVYMELSSLRSEDTAVYYCAR<u>GDLYALDY</u>WGQGTL VTVSS

SEQ ID NO.10 (CDRs: SEQ ID NO.91/92/93): hz18A10-VL

DIQMTQSPSSLSASVGDRVTITC<u>KASQSVSNDVA</u>WYQQKPGKAPKLLIY<u>SASNRYT</u>GVP SRFSGSGSGTDFTFTISSLQPEDIATYYC<u>QHDYSSPYT</u>FGQGTKLEIK

SEQ ID NO.11 (CDRs: SEQ ID NO.94/100/96): hz8C8-VH

EVQLVESGGGLVQPGGSLRLSCAASGFTFS<u>SFGMH</u>WVRQAPGKGLEWVA<u>YITSGSNTI YYADTVKG</u>RFTISRDNAKNSLYLQMNSLRAEDTAVYYCAR<u>EYYSYDEGFAY</u>WGQGTL VTVSS

SEQ ID NO.12 (CDRs: SEQ ID NO.97/98/99): hz8C8-VL

EIVLTQSPATLSLSPGERATLSC<u>SVSSSISSNYLH</u>WYQQKPGQAPRLLIY<u>RTSNLAS</u>GIPAR FSGSGSGTDFTLTISSLEPEDFAVYYC<u>QQGVDVPFT</u>FGQGTKLEIK

According to the amino acid sequences of the humanized antibodies obtained, antibody coding genes were optimized and synthesized. Cleavage sites of restriction enzymes were designed and introduced into both ends of the genes, and the genes encoding the variable regions of the antibodies were linked with the genes encoding the heavy and light chain constant regions of human antibody (the amino acid sequences are as shown in SEQ ID NO. 13 and SEQ ID NO. 14) through enzyme digestion. The obtained genes were cloned into a eukaryotic cell transient expression vector respectively, and expression vectors of recombinant humanized antibodies were obtained. Subsequently, following the method provided in Example 2, recombinant humanized antibody hz18A10 derived from the clone 18A10 was prepared, and recombinant humanized antibody hz8C8 derived from the clone 8C8 was prepared.

The affinity of hz18A10 and hz8C8 was determined according to the method provided in Example 1 using AHC bioprobes for capturing human Fc fragment instead, with other conditions unchanged. A recombinantly expressed anti-IL-11 antibody 3C6-hFc was used at the same concentration as a positive control. Final results of the affinity measurement are shown in Table 4 and Table 7.

The sequences of the positive control anti-IL-11 antibody 3C6-hFc are disclosed in the patent publication WO2019/238882A1. The sequence of the heavy chain variable region of 3C6-hFc is named as "3 C6-VH" and as shown in SEQ ID NO. 1, and the sequence of the light chain variable region of 3C6-hFc is named as "3C6-VL" and as shown in SEQ ID NO.2; and 3C6-hFc has human IgG1 constant regions, i.e., the heavy chain constant region of human antibody as shown in SEQ ID NO. 13 and the light chain constant region of human antibody as shown in SEQ ID NO. 14.

SEQ ID NO. 13 heavy chain constant region of human antibody

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK

SEQ ID NO. 14 light chain constant region of human antibody

RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

## Example 5 Sequence optimization of hz8C8

Random mutations on all or certain of the amino acid residues in the 6 CDRs comprised by the heavy chain variable region (SEQ ID NO. 11) and the light chain variable region (SEQ ID NO.12) of the humanized antibody hz8C8 were performed by yeast display technology, and yeast-displayed single chain antibody (scFv) libraries (Table 2) were constructed. Then, 4-5 rounds of sorting were performed by magnetic bead sorting technology and flow cytometry sorting technology, and mutants having the affinity equivalent to or better than that of the parent antibody were obtained. Information about "hotspot" amino acids was obtained upon a comprehensive analysis of the mutation sites in the mutants; then those hotspot amino acids were recombined and mutant sequences of light and heavy chain variable regions were designed (Table 3) respectively in considering avoiding those sites which might bring immunogenic risks, e.g., T cell epitopes, and B cell epitopes, as well as PTM sites.

Then, light and heavy chain expression vectors for preparing mutant antibodies were constructed according to the method for preparing the recombinant antibodies provided in Example 4, using the mutant sequences shown in Table 3 which accordingly were linked with human IgG1 constant regions (SEQ ID NO. 13, and SEQ ID NO.14). Subsequently, the light chain and the heavy chain were reasonably matched and co-transfected into eukaryotic cells for transient expression, and then recombination protein samples of the mutant antibodies were obtained through affinity purification. The samples obtained were tested for affinity on Fortebio Octet, according to the method for measuring affinity provided in Example 1. Final results of the measurement are shown in Table 4 below. Further, using the protocol provided in Example 3, hz8C8 and its affinity matured mutants were analyzed for their activity for blocking the formation of IL-11/IL-11Rα/GP130 complex. The results are shown in Table 4 below.

SEQ ID NO.94: hz8C8 H-CDR1
SFGMH

SEQ ID NO.100: hz8C8 H-CDR2
YITSGSNTIYYADTVKG

SEQ ID NO.96: hz8C8 H-CDR3
EYYSYDEGFAY

SEQ ID NO.97: hz8C8 L-CDR1
SVSSSISSNYLH

SEQ ID NO.98: hz8C8 L-CDR2
RTSNLAS

SEQ ID NO.99: hz8C8 L-CDR3
QQGVDVPFT

Table 2. Design for constructing random mutation libraries of CDRs in hz8C8

| Name of mutation library | Mutation region | Mutation sites (underlined) | Capacity of mutation library |
|---|---|---|---|
| hz8C8-H1 | H-CDR1 | SFGMH | 5.00E+07 |
| hz8C8-H2 | H-CDR2 | YITSGSNTIYYADTVKG | 1.10E+08 |
| hz8C8-H3 | H-CDR3 | EYYSYDEGFAY | 8.00E+07 |
| hz8C8-L1 | L-CDR1 | SVSSSISSNYLH | 1.30E+08 |
| hz8C8-L2 | L-CDR2 | RTSNLAS | 1.30E+08 |
| hz8C8-L3 | L-CDR3 | QQGVDVPFT | 1.60E+08 |

Table 3. Sequences of mutant antibodies obtained upon optimization of the sequences of hz8C8

| Type | Name | SEQ ID NO. | Sequence (CDRs underlined) |
|---|---|---|---|
| **Heavy chain** | hz8C8-VH (parent) | SEQ ID NO 11 | EVQLVESGGGLVQPGGSLRLSCAASGFTFS**SFGMH**WVRQAPGKGLEWVA**YITSGSNTIYYADTVKG**RFTISRDNAKNSLYLQMNSLRAEDTAVYYCAR**EYYSYDEGFAY**WGQGTLVTVSS (CDRs; SEQ ID NO.94/100/96) |
| | hz8C8-VHm1 | SEQ ID NO.15 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYYSYDEGFAY**WGQGTLVTVSS (CDRs; SEQ ID NO.94/101/96) |
| | hz8C8-VHm2 | SEQ ID NO.16 | EVQLVESGGGLVQPGGSLRLSCAASGFTFS**SFGMH**WVRQAPGKGLEWVA**YITAGSNTIYYADTVKG**RFTISRDNAKNSLYLQMNSLRAEDTAVYYCAR**EYYSYDEGFAY**WGQGTLVTVSS (CDRs; SEQ ID NO.94/102/96) |
| | hz8C8-VHm3 | SEQ ID NO.17 | EVQLVESGGGLVQPGGSLRLSCAASGFTFS**SFGMH**WVRQAPGKGLEWVA**YITPGSNTTYYADTVKG**RFTISRDNAKNSLYLQMNSLRAEDTAVYYCAR**EYYSYDEGFAY**WGQGTLVTVSS (CDRs; SEQ ID NO.94/103/96) |

(continued)

| Type | Name | SEQ ID NO. | Sequence (CDRs underlined) |
|---|---|---|---|
| | hz8C8-VHm4 | SEQ ID NO.18 | EVQLVESGGGLVQPGGSLRLSCAASGFTFS**SFGMH**WVRQAPGKGLEWVA**YITSGANTIYYADTVKG**RFTISRDNAKNSLYLQMNSLRAEDTAVYYCAR**EYYSYDEGFAY**WGQGTLVTVSS<br>(CDRs; SEQ ID NO.94/104/96) |
| | hz8C8-VHm5 | SEQ ID NO.19 | EVQLVESGGGLVQPGGSLRLSCAASGFTFS**SFGMS**WVRQAPGKGLEWVA**YITPGSNTIYYADTVKG**RFTISRDNAKNSLYLQMNSLRAEDTAVYYCAR**EYYSYDEGFAY**WGQGTLVTVSS<br>(CDRs; SEQ ID NO.105/101/96) |
| | hz8C8-VHm6 | SEQ ID NO.20 | EVQLVESGGGLVQPGGSLRLSCAASGFTFS**SFGMH**WVRQAPGKGLEWVA**YITPGSTIYYADTVKG**RFTISRDNAKNSLYLQMNSLRAEDTAVYYCAR**EYYSYDEGFAY**WGQGTLVTVSS<br>(CDRs; SEQ ID NO.94/106/96) |
| | hz8C8-VHm1-m1 | SEQ ID NO.21 | EVQLVESGGGLVQPGGSLRLSCAASGFTFS**SYGMH**WVRQAPGKGLEWVA**YITPGSNTIYYADTVKG**RFTISRDNAKNSLYLQMNSLRAEDTAVYYCAR**EYYSYDEGFAY**WGQGTLVTVSS<br>(CDRs; SEQ ID NO.107/101/96) |
| | hz8C8-VHm1-m2 | SEQ ID NO.22 | EVQLVESGGGLVQPGGSLRLSCAASGFTFS**SFGMH**WVRQAPGKGLEWVA**SITPGSNTIYYADTVKG**RFTISRDNAKNSLYLQMNSLRAEDTAVYYCAR**EYYSYDEGFAY**WGQGTLVTVSS<br>(CDRs; SEQ ID NO.94/108/96) |

(continued)

| Type | Name | SEQ ID NO. | Sequence (CDRs underlined) |
|------|------|-----------|----------------------------|
| | hz8C8-VHm1-m3 | SEQ ID NO.23 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYSSYDEGFAY**WGQGTLVTVSS<br>(CDRs; SEQ ID NO.94/101/109) |
| | hz8C8-VHm1-m4 | SEQ ID NO.24 | EVQLVESGGGLVQPGGSLRLSCAASGFTFS**SFGMH**WVRQAPGKGLEWVS**YITPGSNTIYYADTVKG**RFTISRDNAKNSLYLQMNSLRAEDTAVYYCAR**EYYSYDEGFAY**WGQGTLVTVSS<br>(CDRs; SEQ ID NO.94/101/96) |
| | hz8C8-VHm1-m5 | SEQ ID NO.25 | EVQLVESGGGLVQPGGSLRLSCAASGFTFS**SFGMH**WVRQAPGKGLEYVA**YITPGSNTIYYADTVKG**RFTISRDNAKNSLYLQMNSLRAEDTAVYYCAR**EYYSYDEGFAY**WGQGTLVTVSS<br>(CDRs; SEQ ID NO.94/101/96) |
| | hz8C8-VHm1-m6 | SEQ ID NO.26 | EVQLVESGGGLVQPGGSLRLSCAASGFTFS**SFGMH**WVRQAPGKGLEWVA**VITPGSNTIYYADTVKG**RFTISRDNAKNSLYLQMNSLRAEDTAVYYCAR**EYYSYDEGFAY**WGQGTLVTVSS<br>(CDRs; SEQ ID NO.94/110/96) |
| | hz8C8-VHm1-m7 | SEQ ID NO.27 | EVQLVESGGGLVQPGGSLRLSCAASGFTFS**SFGMH**WVRQAPGKGLEWVS**VITPGSNTIYYADTVKG**RFTISRDNAKNSLYLQMNSLRAEDTAVYYCAR**EYYSYDEGFAY**WGQGTLVTVSS<br>(CDRs; SEQ ID NO.94/110/96) |

(continued)

| Type | Name | SEQ ID NO. | Sequence (CDRs underlined) |
|---|---|---|---|
| | hz8C8-VHm1-m8 | SEQ ID NO.28 | EVQLVESGGGLVQPGGSLRLSCAASGFTFS**SFGMH**WVRQAPGKGLEW**IGYITPGSNTIYYADTVKG**RFTISRDNAKNSLYLQMNSLRAEDTAVYYCAR**EYYSYDEGFAY**WGQGTLVTVSS (CDRs; SEQ ID NO.94/101/96) |
| | hz8C8-VHm1-m9 | SEQ ID NO.29 | EVQLVESGGGLVQPGGSLRLSCAASGFTFS**SFGMH**WVRQAPGKGLEW**MGYITPGSNTIYYADTVKG**RFTISRDNAKNSLYLQMNSLRAEDTAVYYCAR**EYYSYDEGFAY**WGQGTLVTVSS (CDRs; SEQ ID NO.94/101/96) |
| | hz8C8-VHm1-m10 | SEQ ID NO.30 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYYSSDEGFAY**WGQGTLVTVSS (CDRs; SEQ ID NO.94/101/111) |
| | hz8C8-VHm1-m11 | SEQ ID NO.31 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYYSYDEGPAY**WGQGTLVTVSS (CDRs; SEQ ID NO.94/101/112) |
| | hz8C8-VHm1-m12 | SEQ ID NO.32 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYYSYDEGGAY**WGQGTLVTVSS (CDRs; SEQ ID NO.94/101/113) |

(continued)

| Type | Name | SEQ ID NO. | Sequence (CDRs underlined) |
|---|---|---|---|
| | hz8C8-VHm1-m13 | SEQ ID NO.33 | EVQLVESGGGLVQPGGSLRLSCAASGFTFS**SFGMH**WVRQAPGKGLEWVA**YITPGSNTIYYADTVKG**RFTISRDNAKNSLYLQMNSLRAEDTAVYYCAR**EYYSYDEGFAS**WGQGTLVTVSS (CDRs; SEQ ID NO.94/101/114) |
| Light chain | hz8C8-VL (parent) | SEQ ID NO.12 | EIVLTQSPATLSLSPGERATLSC**SVSSSISSNYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.97/98/99) |
| | hz8C8-VLm1 | SEQ ID NO.34 | EIVLTQSPATLSLSPGERATLSC**SVSSSIPSNYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.115/98/99) |
| | hz8C8-VLm2 | SEQ ID NO.35 | EIVLTQSPATLSLSPGERATLSC**SVSSSISSKYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.116/98/99) |
| | hz8C8-VLm3 | SEQ ID NO.36 | EIVLTQSPATLSLSPGERATLSC**SVSSLISSNYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.117/98/99) |
| | hz8C8-VLm4 | SEQ ID NO.37 | EIVLTQSPATLSLSPGERATLSC**SVSDLISSNYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.118/98/99) |

| Type | Name | SEQ ID NO. | Sequence (CDRs underlined) |
|---|---|---|---|
| | hz8C8-VLm5 | SEQ ID NO.38 | EIVLTQSPATLSLSPGERATLSC**SVSELLS TNYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.119/98/99) |
| | hz8C8-VLm6 | SEQ ID NO.39 | EIVLTQSPATLSLSPGERATLSC**SVSSLIPS KYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.120/98/99) |
| | hz8C8-VLm7 | SEQ ID NO.40 | EIVLTQSPATLSLSPGERATLSC**SVSDLIPS QYLH**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.121/98/99) |
| | hz8C8-VLm8 | SEQ ID NO.41 | EIVLTQSPATLSLSPGERATLSC**SVSDLIPS QYLS**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.122/98/99) |
| | hz8C8-VLm9 | SEQ ID NO.42 | EIVLTQSPATLSLSPGERATLSC**SVSDLIPS QYLA**WYQQKPGQAPRLLIY**RTSNLAS**GIPARFSGSGSGTDFTLTISSLEPEDFAVYYC**QQGVDVPFT**FGQGTKLEIK (CDRs; SEQ ID NO.123/98/99) |
| | hz8C8-VLm7-m1 | SEQ ID NO.43 | EIVLTQSPATLSLSPGERATLSC**SVSDLIPS QYLH**WYQQKPGQAPRLLIY**RASNLAS**GIPARFSGSGSGTDFTLTISSLEPEDFAVYYC**QQGVDVPFT**FGQGTKLEIK (CDRs; SEQ ID NO.121/124/99) |

(continued)

| Type | Name | SEQ ID NO. | Sequence (CDRs underlined) |
|---|---|---|---|
| | hz8C8-VLm7-m2 | SEQ ID NO.44 | EIVLTQSPATLSLSPGERATLSC**SVSDLIPS QYLH**WYQQKPGQAPRLLIY**DASNLAS**G IPARFSGSGSGTDFTLTISSLEPEDFAVYY C**QQGVDVPFT**FGQGTKLEIK (CDRs; SEQ ID NO.121/125/99) |
| | hz8C8-VLm7-m3 | SEQ ID NO.45 | EIVLTQSPATLSLSPGERATLSC**SVSDLIPS QYLH**WYQQKPGQAPRLLIY**GASNLAS**G IPARFSGSGSGTDFTLTISSLEPEDFAVYY C**QQGVDVPFT**FGQGTKLEIK (CDRs; SEQ ID NO.121/126/99) |
| | hz8C8-VLm7-m4 | SEQ ID NO.46 | EIVLTQSPATLSLSPGERATLSC**SVSDLIPS QYLH**WYQQKPGQAPRLLIY**RTSNRAS**G IPARFSGSGSGTDFTLTISSLEPEDFAVYY C**QQGVDVPFT**FGQGTKLEIK (CDRs; SEQ ID NO.121/127/99) |

Table 4. Results of the affinity measurement of the mutant antibodies obtained upon optimization of the sequences of hz8C8

| Sample information | | Affinity parameters | | | Blocking activity |
|---|---|---|---|---|---|
| Heavy chain variable region | Light chain variable region | KD (M) | kon (1/Ms) | kdis (1/s) | IC50 ($\mu$g/ml) |
| hz8C8-VHm1 (SEQ ID NO.15) | hz8C8-VL (SEQ ID NO.12) | 6.13E-10 | 7.70E+05 | 4.72E-04 | 2.089 |
| hz8C8-VHm2 (SEQ ID NO.16) | hz8C8-VL (SEQ ID NO.12) | 7.27E-10 | 8.06E+05 | 5.86E-04 | 0.694 |
| hz8C8-VHm3 (SEQ ID NO.17) | hz8C8-VL (SEQ ID NO.12) | 4.77E-09 | 4.53E+05 | 2.16E-03 | 0.828 |
| hz8C8-VHm4 (SEQ ID NO.18) | hz8C8-VL (SEQ ID NO.12) | 1.37E-09 | 8.78E+05 | 1.20E-03 | 1.01 |
| hz8C8-VHm1 (SEQ ID NO.15) | hz8C8-VLm1 (SEQ ID NO.34) | 1.78E-10 | 7.05E+05 | 1.25E-04 | 1.241 |
| hz8C8-VHm1 (SEQ ID NO.15) | hz8C8-VLm2 (SEQ ID NO.35) | 3.88E-10 | 5.90E+05 | 2.29E-04 | 1.14 |
| hz8C8-VHm1 (SEQ ID NO.15) | hz8C8-VLm3 (SEQ ID NO.36) | 6.36E-10 | 7.80E+05 | 4.96E-04 | 0.943 |
| hz8C8-VHm1 (SEQ ID NO.15) | hz8C8-VLm4 (SEQ ID NO.37) | 1.61E-10 | 8.74E+05 | 1.41E-04 | 0.846 |

(continued)

| Sample information | | Affinity parameters | | | Blocking activity |
|---|---|---|---|---|---|
| Heavy chain variable region | Light chain variable region | KD (M) | kon (1/Ms) | kdis (1/s) | IC50 (μg/ml) |
| hz8C8-VHm1 (SEQ ID NO.15) | hz8C8-VLm5 (SEQ ID NO.38) | 4.79E-10 | 8.48E+05 | 4.06E-04 | 0.838 |
| hz8C8-VHm1 (SEQ ID NO.15) | hz8C8-VLm6 (SEQ ID NO.39) | 4.12E-11 | 5.43E+05 | 2.23E-05 | 1.011 |
| hz8C8-VHm1 (SEQ ID NO.15) | hz8C8-VLm7 (SEQ ID NO.40) | 4.29E-12 | 8.22E+05 | 3.52E-06 | 0.85 |
| hz8C8-VHm1 (SEQ ID NO.15) | hz8C8-VLm8 (SEQ ID NO.41) | 3.13E-11 | 9.48E+05 | 2.97E-05 | 0.7 |
| hz8C8-VHm1 (SEQ ID NO.15) | hz8C8-VLm9 (SEQ ID NO.42) | 3.54E-11 | 9.75E+05 | 3.45E-05 | 0.72 |
| hz8C8-VHm5 (SEQ ID NO.19) | hz8C8-VLm7 (SEQ ID NO.40) | 1.90E-10 | 9.20E+05 | 1.75E-04 | 0.65 |
| hz8C8-VHm6 (SEQ ID NO.20) | hz8C8-VLm7 (SEQ ID NO.40) | 1.56E-10 | 8.12E+05 | 1.27E-04 | 0.54 |
| hz8C8-VHm1-m1 (SEQ ID NO.21) | hz8C8-VLm7 (SEQ ID NO.40) | 1.25E-10 | 1.04E+06 | 1.30E-04 | 0.75 |
| hz8C8-VHm1-m2 (SEQ ID NO.22) | hz8C8-VLm7 (SEQ ID NO.40) | 3.41E-09 | 1.04E+06 | 3.55E-03 | 1.68 |
| hz8C8-VHm1-m3 (SEQ ID NO.23) | hz8C8-VLm7 (SEQ ID NO.40) | 9.87E-11 | 1.12E+06 | 1.10E-04 | 0.83 |
| hz8C8-VHm1 (SEQ ID NO.15) | hz8C8-VLm7-m1 (SEQ ID NO.43) | 1.30E-10 | 1.05E+06 | 1.36E-04 | 0.58 |
| hz8C8-VHm1 (SEQ ID NO.15) | hz8C8-VLm7-m2 (SEQ ID NO.44) | 3.47E-11 | 1.07E+06 | 3.70E-05 | 0.74 |
| hz8C8-VHm1 (SEQ ID NO.15) | hz8C8-VLm7-m3 (SEQ ID NO.45) | 7.95E-11 | 1.13E+06 | 8.99E-05 | 0.73 |
| hz8C8-VHm1 (SEQ ID NO.15) | hz8C8-VLm7-m4 (SEQ ID NO.46) | 1.78E-10 | 1.00E+06 | 1.78E-04 | 0.69 |
| hz8C8-VHm1-m3 (SEQ ID NO.23) | hz8C8-VLm7-m1 (SEQ ID NO.43) | 6.09E-11 | 9.54E+05 | 5.81E-05 | 0.55 |
| hz8C8-VHm1-m3 (SEQ ID NO.23) | hz8C8-VLm7-m2 (SEQ ID NO.44) | <1.0E-12 | 9.45E+05 | <1.0E-07 | 0.67 |
| hz8C8-VHm1-m3 (SEQ ID NO.23) | hz8C8-VLm7-m3 (SEQ ID NO.45) | <1.0E-12 | 9.72E+05 | <1.0E-07 | 0.78 |
| hz8C8-VHm1-m3 (SEQ ID NO.23) | hz8C8-VLm7-m4 (SEQ ID NO.46) | <1.0E-12 | 9.01E+05 | <1.0E-07 | 0.47 |
| hz8C8- VHm1- m4 (SEQ ID NO.24) | hz8C8-VLm7-m2 (SEQ ID NO.44) | <1.0E-12 | 8.79E+05 | <1.0E-07 | 0.79 |
| hz8C8-VHm1-m5 (SEQ ID NO.25) | hz8C8-VLm7-m2 (SEQ ID NO.44) | 5.94E-11 | 6.83E+05 | 4.05E-05 | 0.52 |
| hz8C8-VHm1-m6 (SEQ ID NO.26) | hz8C8-VLm7-m2 (SEQ ID NO.44) | 2.07E-09 | 6.53E+05 | 1.35E-03 | 1.71 |

(continued)

| Sample information | | Affinity parameters | | | Blocking activity |
|---|---|---|---|---|---|
| Heavy chain variable region | Light chain variable region | KD (M) | kon (1/Ms) | kdis (1/s) | IC50 (µg/ml) |
| hz8C8-VHm1-m7 (SEQ ID NO.27) | hz8C8-VLm7-m2 (SEQ ID NO.44) | 1.87E-09 | 5.95E+05 | 1.11E-03 | 2.14 |
| hz8C8-VHm1-m8 (SEQ ID NO.28) | hz8C8-VLm7-m2 (SEQ ID NO.44) | <1.0E-12 | 8.47E+05 | <1.0E-07 | 0.57 |
| hz8C8-VHm1-m9 (SEQ ID NO.29) | hz8C8-VLm7-m2 (SEQ ID NO.44) | 2.39E-11 | 8.97E+05 | 2.14E-05 | 0.77 |
| hz8C8-VHm1-m10 (SEQ ID NO.30) | hz8C8-VLm7-m2 (SEQ ID NO.44) | 1.39E-09 | 2.19E+05 | 3.05E-04 | 1.57 |
| hz8C8-VHm1-m11 (SEQ ID NO.31) | hz8C8-VLm7-m2 (SEQ ID NO.44) | 1.30E-09 | 7.71E+05 | 1.00E-03 | 1.703 |
| hz8C8-VHm1-m12 (SEQ ID NO.32) | hz8C8-VLm7-m2 (SEQ ID NO.44) | 7.95E-09 | 1.75E+05 | 1.39E-03 | 1.54 |
| hz8C8-VHm1-m13 (SEQ ID NO.33) | hz8C8-VLm7-m2 (SEQ ID NO.44) | 5.73E-11 | 7.53E+05 | 4.32E-05 | 0.54 |
| Parent antibody: hz8C8 (SEQ ID NO.11 + SEQ ID NO.12) | | 1.60E-09 | 8.36E+05 | 1.34E-03 | 2.561 |
| Positive control antibody: 3C6-hFc (SEQ ID NO.1 + SEQ ID NO.2) | | 7.12E-10 | 8.16E+05 | 5.81E-04 | 1.9 |

## Example 6 Sequence optimization of hz18A10

Following the protocol for performing affinity maturation of hz8C8 provided above, mutation libraries (Table 5) were constructed by a yeast display technology, and used for performing affinity maturation and sequence optimization of the humanized anti-IL-11 antibody hz18A10. Mutant sequences of light and heavy chain variable regions designed were shown in Table 6. Mutant antibodies were recombinantly expressed and their affinity was measured which results are shown in Table 7 below.

SEQ ID NO.88: hz18A10 H-CDR1
TYNMD

SEQ ID NO.89: hz18A10 H-CDR2
TIYPGNGDTSYNQKFKG

SEQ ID NO.90: hz18A10 H-CDR3
GDLYALDY

SEQ ID NO.91: hz18A10 L-CDR1
KASQSVSNDVA

SEQ ID NO.92: hz18A10 L-CDR2
SASNRYT

SEQ ID NO.93: hz18A10 L-CDR3
QHDYSSPYT

Table 5. Design for constructing random mutation libraries of CDRs and essential amino acids in neighboring region in hz18A10

| Name of mutation library | Mutation region | Mutation sites (underlined) | Capacity of mutation library |
|---|---|---|---|
| hz18A10-H1 | H-CDR1 | TTYNMD | 6.00E+07 |
| hz18A10-H2 | H-CDR2 | TTYPGNGDTSYNQKFKG | 5.00E+07 |
| hz18A10-H3 | H-CDR3 | GDLYALDY | 2.00E+07 |
| hz18A10-L1 | L-CDR1 | KASQSVSNDVA | 4.00E+07 |
| hz18A10-L2 | L-CDR2 | SASNRYT | 2.00E+07 |
| hz18A10-L3 | L-CDR3 | QHDYSSPYT | 3.00E+07 |

Table 6. Sequences of mutant antibodies obtained upon optimization of the sequences of hz18A10

| Type | Name | SEQ ID NO. | Sequence (CDRs underlined) |
|---|---|---|---|
| **Heavy chain** | hz18A10-VH (parent) | SEQ ID NO.9 | EVQLVQSGAEVKKPGASVKVSCKASGYTFT<u>TYNMD</u>WVRQAPGQGLEWMG<u>TIYPGNGDTSYNQKFKG</u>RVTMTRDTSTSTVYMELSSLRSEDTAVYYCAR<u>GDLYALDY</u>WGQGTLVTVSS (CDRs: SEQ ID NO.88/89/90) |
| | hz18A10-VHm 1 | SEQ ID NO.49 | EVQLVQSGAEVKKPGASVKVSCKASGYTFT**<u>TYNMD</u>**WVRQAPGQGLEWMG**<u>TIYPGNGDTSYNQKFKG</u>**RVTMTRDTSTSTVYMELSSLRSEDTAVYYCAR**<u>GDLHFLDY</u>**WGQGTLVTVSS (CDRs: SEQ ID NO.88/89/130) |
| | hz18A10-VHm2 | SEQ ID NO.50 | EVQLVQSGAEVKKPGASVKVSCKASGYTFT**<u>TYNMD</u>**WVRQAPGQGLEWMG**<u>TIYPGNGDTSYNQKFKG</u>**RVTMTRDTSTSTVYMELSSLRSEDTAVYYCAR**<u>GDLHYLDY</u>**WGQGTLVTVSS (CDRs: SEQ ID NO.88/89/131) |
| | hz18A10-VHm3 | SEQ ID NO.51 | EVQLVQSGAEVKKPGASVKVSCKASGYTFT**<u>TYDMD</u>**WVRQAPGQGLEWMG**<u>TIYPGNGDTSYNQKFKG</u>**RVTMTRDTSTSTVYMELSSLRSEDTAVYYCAR**<u>GDLYALDY</u>**WGQGTLVTVSS (CDRs: SEQ ID NO.132/89/90) |

| Type | Name | SEQ ID NO. | Sequence (CDRs underlined) |
|---|---|---|---|
| | hz18A10-VHm4 | SEQ ID NO.52 | EVQLVQSGAEVKKPGASVKVSCKASGY TFT**TYNID**WVRQAPGQGLEWMG**TIYPG NGDTSYNQKFKG**RVTMTRDTSTSTVY MELSSLRSEDTAVYYCAR**GDLYALDY**W GQGTLVTVSS (CDRs: SEQ ID NO.133/89/90) |
| | hz18A10-VHm5 | SEQ ID NO.53 | EVQLVQSGAEVKKPGASVKVSCKASGY TFI**TYNMD**WVRQAPGQGLEWMG**TIYP GNGDTSYNQKFKG**RVTMTRDTSTSTAY MELSSLRSEDTAVYYCAR**ADLHFLDY**W GQGTLVTVSS (CDRs: SEQ ID NO.88/89/134) |
| | hz18A10-VHm6 | SEQ ID NO.54 | EVQLVQSGAEVKKPGASVKVSCKASGY TFN**HYDMD**WVRQAPGQGLEWMG**TIYP GNGDTSYNQKFKG**RVTMTRDTSTSTVY MELSSLRSEDTAVYYCAR**GDLYALDY**W GQGTLVTVSS (CDRs: SEQ ID NO.135/89/90) |
| | hz18A10-VHm7 | SEQ ID NO.55 | EVQLVQSGAEVKKPGASVKVSCKASGY TFT**TYDID**WVRQAPGQGLEWMG**TIYPG NGDTSYNQKFKG**RVTMTRDTSTSTVY MELSSLRSEDTAVYYCAR**GDLYALDY**W GQGTLVTVSS (CDRs: SEQ ID NO.136/89/90) |
| | hz18A10-VHm8 | SEQ ID NO.56 | EVQLVQSGAEVKKPGASVKVSCKASGY TFT**TYDMH**WVRQAPGQGLEWMG**TIYP GNGDTSYNQKFKG**RVTMTRDTSTSTVY MELSSLRSEDTAVYYCAR**GDLYALDY**W GQGTLVTVSS (CDRs: SEQ ID NO.137/89/90) |

(continued)

| Type | Name | SEQ ID NO. | Sequence (CDRs underlined) |
|---|---|---|---|
| | hz18A10-VHm9 | SEQ ID NO.57 | EVQLVQSGAEVKKPGASVKVSCKASGYTFT**TYDMS**WVRQAPGQGLEWMG**TIYPGNGDTSYNQKFKG**RVTMTRDTSTSTVYMELSSLRSEDTAVYYCAR**GDLYALDY**WGQGTLVTVSS (CDRs：SEQ ID NO.138/89/90) |
| | hz18A10-VHm10 | SEQ ID NO.58 | EVQLVQSGAEVKKPGASVKVSCKASGYTFN**HYNMD**WVRQAPGQGLEWMG**TIYPGNGDTSYNQKFKG**RVTMTRDTSTSTVYMELSSLRSEDTAVYYCAR**GDLYALDY**WGQGTLVTVSS (CDRs：SEQ ID NO.139/89/90) |
| | hz18A10-VHm11 | SEQ ID NO.59 | EVQLVQSGAEVKKPGASVKVSCKASGYTFT**HYNMD**WVRQAPGQGLEWMG**TIYPGNGDTSYNQKFKG**RVTMTRDTSTSTVYMELSSLRSEDTAVYYCAR**GDLYALDY**WGQGTLVTVSS (CDRs：SEQ ID NO.139/89/90) |
| | hz18A10-VHm12 | SEQ ID NO.60 | EVQLVQSGAEVKKPGASVKVSCKASGYTFT**HYDMD**WVRQAPGQGLEWMG**TIYPGNGDTSYNQKFKG**RVTMTRDTSTSTVYMELSSLRSEDTAVYYCAR**GDLYALDY**WGQGTLVTVSS (CDRs：SEQ ID NO.135/89/90) |
| | hz18A10-VHm13 | SEQ ID NO.61 | EVQLVQSGAEVKKPGASVKVSCKASGYTFT**HYEMD**WVRQAPGQGLEWMG**TIYPGNGDTSYNQKFKG**RVTMTRDTSTSTVYMELSSLRSEDTAVYYCAR**GDLYALDY**WGQGTLVTVSS (CDRs：SEQ ID NO.140/89/90) |

(continued)

| Type | Name | SEQ ID NO. | Sequence (CDRs underlined) |
|---|---|---|---|
| | hz18A10-VHm14 | SEQ ID NO.62 | EVQLVQSGAEVKKPGASVKVSCKASGYTFT**HYQMD**WVRQAPGQGLEWMG**TIYPGNGDTSYNQKFKG**RVTMTRDTSTSTVYMELSSLRSEDTAVYYCAR**GDLYALDY**WGQGTLVTVSS<br>(CDRs：SEQ ID NO.141/89/90) |
| | hz18A10-VHm15 | SEQ ID NO.63 | EVQLVQSGAEVKKPGASVKVSCKASGYTFT**HYSMD**WVRQAPGQGLEWMG**TIYPGNGDTSYNQKFKG**RVTMTRDTSTSTVYMELSSLRSEDTAVYYCAR**GDLYALDY**WGQGTLVTVSS<br>(CDRs：SEQ ID NO.142/89/90) |
| | hz18A10-VHm16 | SEQ ID NO.64 | EVQLVQSGAEVKKPGASVKVSCKASGYTFT**HYDMD**WVRQAPGQGLEWMG**TIYPGNADTSYNQKFKG**RVTMTRDTSTSTVYMELSSLRSEDTAVYYCAR**GDLYALDY**WGQGTLVTVSS<br>(CDRs：SEQ ID NO.135/143/90) |
| | hz18A10-VHm17 | SEQ ID NO.65 | EVQLVQSGAEVKKPGASVKVSCKASGYTFT**HYDMD**WVRQAPGQGLEWMG**TIYPGEGDTSYNQKFKG**RVTMTRDTSTSTVYMELSSLRSEDTAVYYCAR**GDLYALDY**WGQGTLVTVSS<br>(CDRs：SEQ ID NO.135/144/90) |
| | hz18A10-VHm18 | SEQ ID NO.66 | EVQLVQSGAEVKKPGASVKVSCKASGYTFT**HYNMD**WVRQAPGQGLEWMG**TIYPGEGDTSYNQKFKG**RVTMTRDTSTSTVYMELSSLRSEDTAVYYCAR**GDLYALDY**WGQGTLVTVSS<br>(CDRs：SEQ ID NO.139/144/90) |

| Type | Name | SEQ ID NO. | Sequence (CDRs underlined) |
|---|---|---|---|
| | hz18A10-VHm19 | SEQ ID NO.67 | EVQLVQSGAEVKKPGASVKVSCKASGYTFT**HYNMD**WVRQAPGQGLEWMG**TIYPGNADTSYNQKFKG**RVTMTRDTSTSTVYMELSSLRSEDTAVYYCAR**GDLYALDY**WGQGTLVTVSS (CDRs：SEQ ID NO.139/143/90) |
| | hz18A10-VHm20 | SEQ ID NO.68 | EVQLVQSGAEVKKPGASVKVSCKASGYTFT**HYNMD**WVRQAPGQGLEWMG**TIYPGQGDTSYNQKFKG**RVTMTRDTSTSTVYMELSSLRSEDTAVYYCAR**GDLYALDY**WGQGTLVTVSS (CDRs：SEQ ID NO.139/145/90) |
| **Light chain** | hz18A10-VL (parent) | SEQ ID NO.10 | DIQMTQSPSSLSASVGDRVTITCKASQSVSNDVAWYQQKPGKAPKLLIYSASNRYTGVPSRFSGSGSGTDFTFTISSLQPEDIATYYCQHDYSSPYTFGQGTKLEIK (CDRs：SEQ ID NO.91/92/93) |
| | hz18A10-VLm1 | SEQ ID NO.69 | DIQMTQSPSSLSASVGDRVTITC**KASKSVSNDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.146/92/93) |
| | hz18A10-VLm2 | SEQ ID NO.70 | DIQMTQSPSSLSASVGDRVTITC**KASYSVSNDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.147/92/93) |

(continued)

| Type | Name | SEQ ID NO. | Sequence (CDRs underlined) |
|---|---|---|---|
| | hz18A10-VLm3 | SEQ ID NO.71 | DIQMTQSPSSLSASVGDRVTITC**KASHSVSNDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.148/92/93) |
| | hz18A10-VLm4 | SEQ ID NO.72 | DIQMTQSPSSLSASVGDRVTITC**KASQFVSNDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.149/92/93) |
| | hz18A10-VLm5 | SEQ ID NO.73 | DIQMTQSPSSLSASVGDRVTITC**KASQSVSNDIA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.150/92/93) |
| | hz18A10-VLm6 | SEQ ID NO.74 | DIQMTQSPSSLSASVGDRVTITC**KASQSVFHDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.151/92/93) |
| | hz18A10-VLm7 | SEQ ID NO.75 | DIQMTQSPSSLSASVGDRVTITC**KASQSVFRDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.152/92/93) |
| | hz18A10-VLm8 | SEQ ID NO.76 | DIQMTQSPSSLSASVGDRVTITC**KASQSVYHDVA**WYQQKPGKAPKLLIY**SASNRYT**GVPSRFSGSGSGTDFTFTISSLQPEDIATYYC**QHDYSSPYT**FGQGTKLEIK (CDRs：SEQ ID NO.153/92/93) |

(continued)

| Type | Name | SEQ ID NO. | Sequence (CDRs underlined) |
|---|---|---|---|
| | hz18A10-VLm9 | SEQ ID NO.77 | DIQMTQSPSSLSASVGDRVTITC**KASQSVYSDVA**WYQQKPGKAPKLLIY**SASNRYT**GVPSRFSGSGSGTDFTFTISSLQPEDIATYC**QHDYSSPYT**FGQGTKLEIK (CDRs：SEQ ID NO.154/92/93) |
| | hz18A10-VLm10 | SEQ ID NO.78 | DIQMTQSPSSLSASVGDRVTITC**KASQSVSNDLA**WYQQKPGKAPKLLIY**SASNRYT**GVPSRFSGSGSGTDFTFTISSLQPEDIATYC**QHDYSSPYT**FGQGTKLEIK (CDRs：SEQ ID NO.155/92/93) |
| | hz18A10-VLm11 | SEQ ID NO.79 | DIQMTQSPSSLSASVGDRVTITC**KASQSVSNDVA**WYQQKPGKAPKLLIY**RASNRYT**GVPSRFSGSGSGTDFTFTISSLQPEDIATYC**QHDYSSPYT**FGQGTKLEIK (CDRs：SEQ ID NO.91/156/93) |
| | hz18A10-VLm12 | SEQ ID NO.80 | DIQMTQSPSSLSASVGDRVTITC**KASQSVSNDVA**WYQQKPGKAPKLLIY**SASSRYT**GVPSRFSGSGSGTDFTFTISSLQPEDIATYC**QHDYSSPYT**FGQGTKLEIK (CDRs：SEQ ID NO.91/157/93) |
| | hz18A10-VLm13 | SEQ ID NO.81 | DIQMTQSPSSLSASVGDRVTITC**KASQSVSNDVA**WYQQKPGKAPKLLIY**SASRRYT**GVPSRFSGSGSGTDFTFTISSLQPEDIATYC**QHDYSSPYT**FGQGTKLEIK (CDRs：SEQ ID NO.91/158/93) |
| | hz18A10-VLm14 | SEQ ID NO.82 | DIQMTQSPSSLSASVGDRVTITC**KASQSVSNDVA**WYQQKPGKAPKLLIY**RASRRYT**GVPSRFSGSGSGTDFTFTISSLQPEDIATYC**QHDYSSPYT**FGQGTKLEIK (CDRs：SEQ ID NO.91/159/93) |

(continued)

| Type | Name | SEQ ID NO. | Sequence (CDRs underlined) |
|---|---|---|---|
| | hz18A10-VLm15 | SEQ ID NO.83 | DIQMTQSPSSLSASVGDRVTITC**KASQSVSNDVA**WYQQKPGKAPKLLIY**RASSRYT**GVPSRFSGSGSGTDFTFTISSLQPEDIATYC**QHDYSSPYT**FGQGTKLEIK (CDRs: SEQ ID NO.91/160/93) |
| | hz18A10-VLm16 | SEQ ID NO.84 | DIQMTQSPSSLSASVGDRVTITC**KASQSVSNDVA**WYQQKPGKAPKLLIY**RASRRYP**GVPSRFSGSGSGTDFTFTISSLQPEDIATYC**QHDYSSPYT**FGQGTKLEIK (CDRs: SEQ ID NO.91/161/93) |
| | hz18A10-VLm17 | SEQ ID NO.85 | DIQMTQSPSSLSASVGDRVTITC**KASQSVYRDVA**WYQQKPGKAPKLLIY**RASNRYT**GVPSRFSGSGSGTDFTFTISSLQPEDIATYC**QHDYSSPYT**FGQGTKLEIK (CDRs: SEQ ID NO.162/156/93) |
| | hz18A10-VLm18 | SEQ ID NO.86 | DIQMTQSPSSLSASVGDRVTITC**KASQSVYRDVA**WYQQKPGKAPKLLIY**RASSRYT**GVPSRFSGSGSGTDFTFTISSLQPEDIATYC**QHDYSSPYT**FGQGTKLEIK (CDRs: SEQ ID NO.162/160/93) |
| | hz18A10-VLm19 | SEQ ID NO.87 | DIQMTQSPSSLSASVGDRVTITC**KASQSVYRDVA**WYQQKPGKAPKLLIY**SASNRYT**GVPSRFSGSGSGTDFTFTISSLQPEDIATYC**QHDYSSPYT**FGQGTKLEIK (CDRs: SEQ ID NO.162/92/93) |

Table 7. Results of the affinity measurement of the mutant antibodies obtained upon optimization of the sequences of hz18A10

| Sample information | | Affinity parameters | | |
|---|---|---|---|---|
| Heavy chain variable region | Light chain variable region | KD (M) | kon (1/Ms) | kdis (1/s) |
| hz18A10-VH (SEQ ID NO.9) | hz18A10-VL (SEQ ID NO.10) | 3.21E-09 | 7.73E+05 | 2.49E-03 |
| hz18A10-VH (SEQ ID NO.9) | hz18A10-VLm1 (SEQ ID NO.69) | 4.61E-09 | 7.18E+05 | 3.31E-03 |
| hz18A10-VH (SEQ ID NO.9) | hz18A10-VLm2 (SEQ ID NO.70) | 2.30E-09 | 8.06E+05 | 1.85E-03 |

(continued)

| Sample information | | Affinity parameters | | |
| --- | --- | --- | --- | --- |
| Heavy chain variable region | Light chain variable region | KD (M) | kon (1/Ms) | kdis (1/s) |
| hz18A10-VH (SEQ ID NO.9) | hz18A10-VLm3 (SEQ ID NO.71) | 3.94E-09 | 7.04E+05 | 2.77E-03 |
| hz18A10-VH (SEQ ID NO.9) | hz18A10-VLm4 (SEQ ID NO.72) | 3.12E-09 | 7.60E+05 | 2.37E-03 |
| hz18A10-VH (SEQ ID NO.9) | hz18A10-VLm5 (SEQ ID NO.73) | 4.09E-09 | 6.88E+05 | 2.81E-03 |
| hz18A10-VH (SEQ ID NO.9) | hz18A10-VLm6 (SEQ ID NO.74) | 8.77E-10 | 9.05E+05 | 7.94E-04 |
| hz18A10-VH (SEQ ID NO.9) | hz18A10-VLm8 (SEQ ID NO.76) | 8.03E-10 | 9.29E+05 | 7.46E-04 |
| hz18A10-VH (SEQ ID NO.9) | hz18A10-VLm9 (SEQ ID NO.77) | 5.40E-10 | 8.59E+05 | 4.63E-04 |
| hz18A10-VH (SEQ ID NO.9) | hz18A10-VLm10 (SEQ ID NO.78) | 4.06E-09 | 7.27E+05 | 2.95E-03 |
| hz18A10-VH (SEQ ID NO.9) | hz18A10- VLm11 (SEQ ID NO.79) | 1.80E-09 | 7.47E+05 | 1.34E-03 |
| hz18A10-VH (SEQ ID NO.9) | hz18A10-VLm12 (SEQ ID NO.80) | 3.54E-09 | 7.50E+05 | 2.65E-03 |
| hz18A10-VH (SEQ ID NO.9) | hz18A10-VLm13 (SEQ ID NO.81) | 3.99E-09 | 6.73E+05 | 2.68E-03 |
| hz18A10-VHm1 (SEQ ID NO.49) | hz18A10-VL (SEQ ID NO.10) | 2.17E-09 | 6.40E+05 | 1.39E-03 |
| hz18A10-VHm2 (SEQ ID NO.50) | hz18A10-VL (SEQ ID NO.10) | 2.29E-09 | 6.92E+05 | 1.58E-03 |
| hz18A10-VHm1 (SEQ ID NO.49) | hz18A10-VLm7 (SEQ ID NO.75) | 2.40E-09 | 5.44E+05 | 1.30E-03 |
| hz18A10-VHm1 (SEQ ID NO.49) | hz18A10-VLm14 (SEQ ID NO.82) | 1.03E-09 | 3.30E+05 | 3.41E-04 |
| hz18A10-VHm1 (SEQ ID NO.49) | hz18A10-VLm15 (SEQ ID NO.83) | 5.85E-10 | 4.53E+05 | 2.65E-04 |
| hz18A10-VHm1 (SEQ ID NO.49) | hz18A10-VLm9 (SEQ ID NO.77) | 2.01E-09 | 4.79E+05 | 9.63E-04 |
| hz18A10-VHm1 (SEQ ID NO.49) | hz18A10- VLm11 (SEQ ID NO.79) | 8.25E-10 | 3.48E+05 | 2.87E-04 |
| h18A10-VHm2 (SEQ ID NO.50) | hz18A10-VLm9 (SEQ ID NO.77) | 2.33E-09 | 4.04E+05 | 9.41E-04 |
| hz18A10-VHm2 (SEQ ID NO.50) | hz18A10- VLm11 (SEQ ID NO.79) | 1.01E-09 | 3.14E+05 | 3.17E-04 |
| hz18A10-VHm5 (SEQ ID NO.53) | hz18A10-VLm9 (SEQ ID NO.77) | 1.15E-09 | 6.39E+05 | 7.32E-04 |
| hz18A10-VH (SEQ ID NO.9) | hz18A10-VLm16 (SEQ ID NO.84) | 1.97E-09 | 6.99E+05 | 9.38E-04 |
| hz18A10-VH (SEQ ID NO.9) | hz18A10-VLm17 (SEQ ID NO.85) | 4.50E-10 | 7.25E+05 | 3.26E-04 |
| hz18A10-VHm3 (SEQ ID NO.51) | hz18A10-VLm9 (SEQ ID NO.77) | 9.59E-11 | 1.01E+06 | 9.70E-05 |
| hz18A10-VHm4 (SEQ ID NO.52) | hz18A10-VLm9 (SEQ ID NO.77) | 4.88E-10 | 1.02E+06 | 4.96E-04 |
| hz18A10-VHm6 (SEQ ID NO.54) | hz18A10-VLm9 (SEQ ID NO.77) | 7.18E-12 | 9.99E+05 | 7.17E-06 |
| hz18A10-VH (SEQ ID NO.9) | hz18A10-VLm18 (SEQ ID NO.86) | 4.19E-10 | 7.16E+05 | 3.00E-04 |
| hz18A10-VHm4 (SEQ ID NO.52) | hz18A10-VLm18 (SEQ ID NO.86) | 3.22E-10 | 8.26E+05 | 2.66E-04 |
| hz18A10-VHm7 (SEQ ID NO.55) | hz18A10-VLm19 (SEQ ID NO.87) | 1.05E-10 | 1.08E+06 | 1.13E-04 |
| hz18A10-VHm8 (SEQ ID NO.56) | hz18A10-VLm19 (SEQ ID NO.87) | 1.44E-09 | 7.60E+05 | 1.10E-03 |
| hz18A10-VHm9 (SEQ ID NO.57) | hz18A10-VLm19 (SEQ ID NO.87) | 5.38E-10 | 8.33E+05 | 4.48E-04 |
| hz18A10-VHm3 (SEQ ID NO.51) | hz18A10-VLm19 (SEQ ID NO.87) | 6.83E-10 | 9.54E+05 | 6.52E-04 |
| hz18A10-VHm6 (SEQ ID NO.54) | hz18A10-VLm19 (SEQ ID NO.87) | 2.13E-10 | 9.11E+05 | 1.94E-04 |
| hz18A10-VHm10 (SEQ ID NO.58) | hz18A10-VLm19 (SEQ ID NO.87) | 2.35E-10 | 9.17E+05 | 2.16E-04 |
| hz18A10-VHm11 (SEQ ID NO.59) | hz18A10-VLm19 (SEQ ID NO.87) | 1.38E-10 | 8.62E+05 | 1.19E-04 |
| hz18A10-VHm12 (SEQ ID NO.60) | hz18A10-VLm19 (SEQ ID NO.87) | 1.85E-10 | 9.34E+05 | 1.73E-04 |
| hz18A10-VHm13 (SEQ ID NO.61) | hz18A10-VLm19 (SEQ ID NO.87) | 2.03E-10 | 7.29E+05 | 1.48E-04 |
| hz18A10-VHm14 (SEQ ID NO.62) | hz18A10-VLm19 (SEQ ID NO.87) | 6.78E-10 | 7.16E+05 | 4.85E-04 |
| hz18A10-VHm15 (SEQ ID NO.63) | hz18A10-VLm19 (SEQ ID NO.87) | 3.46E-10 | 7.60E+05 | 2.63E-04 |

(continued)

| Sample information | | Affinity parameters | | |
|---|---|---|---|---|
| Heavy chain variable region | Light chain variable region | KD (M) | kon (1/Ms) | kdis (1/s) |
| hz18A10-VHm16 (SEQ ID NO.64) | hz18A10-VLm19 (SEQ ID NO.87) | 9.56E-11 | 1.26E+06 | 1.20E-04 |
| hz18A10-VHm17 (SEQ ID NO.65) | hz18A10-VLm19 (SEQ ID NO.87) | 7.08E-12 | 1.16E+06 | 8.23E-06 |
| hz18A10-VHm18 (SEQ ID NO.66) | hz18A10-VLm19 (SEQ ID NO.87) | 3.13E-10 | 1.13E+06 | 3.54E-04 |
| hz18A10-VHm19 (SEQ ID NO.67) | hz18A10-VLm19 (SEQ ID NO.87) | 1.01E-10 | 9.93E+05 | 1.00E-04 |
| hz18A10-VHm20 (SEQ ID NO.68) | hz18A10-VLm19 (SEQ ID NO.87) | 2.20E-10 | 1.03E+06 | 2.27E-04 |
| Positive control antibody: 3C6-hFc (SEQ ID NO.1 + SEQ ID NO.2) | | 7.12E-10 | 8.16E+05 | 5.81E-04 |

**Example 7 Analysis of the effect of hz8C8 and its mutants on STAT3 signaling pathway using a reporter gene system**

**(1) Construction of IL-11Rα/GP130/STAT3-luc HEK293 reporter gene system**

To analyze the cytological activity of the antibodies, an IL-11Rα/STAT3-luc HEK293 reporter system was constructed. A vector containing STAT3-Luciference reporter gene system (Cobioer Inc.) was transfected into HEK293 cells, and upon pressurized screening, a stable cell line named as STAT3-Luc/HEK293 which was integrated with the STAT3-Luciference gene was obtained. Next, on the basis of the cell line, a vector containing a full length gene of human IL-11Rα (Sino Biological Inc.) was transfected into the cells and upon pressurized screening, a pool capable of stably expressing human IL-11Rα (IL-11Rα/STAT3-luc HEK293 Pool) was obtained. Finally, separation and screening of monoclones were carried out on the pool to obtain IL-11Rα/STAT3-luc HEK293 monoclonal cells. Those monoclonal cells could stably passage were selected and stored as a cell strain. HEK293 cells constitutively express GP130, so with the stably transfected IL-11Rα/STAT3-luc, an IL-11Rα/GP130/STAT3-luc HEK293 reporter gene system was constituted.

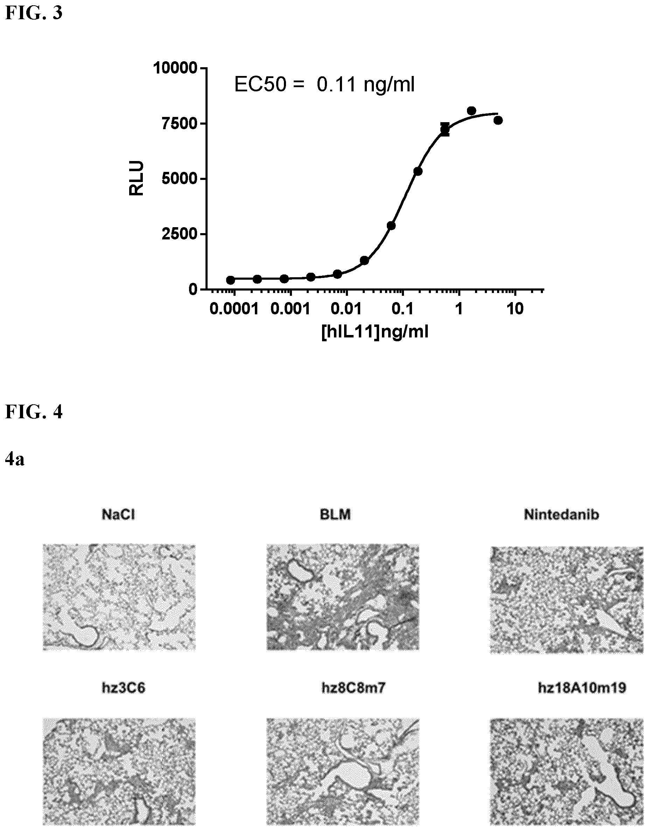

To detect the stimulatory activity, the cells were inoculated in 96-well plates; and IL-11 was diluted to an appropriate concentration and then was subjected to gradient dilution. Then the diluted IL-11 was added into the cells in the 96-well plates, in which blank wells were also set. When the data was processed, fluorescence measurements from the blank wells were deducted from those of all the other wells, and a dose-effect curve was established using the obtained data. The results (FIG. 3) showed that IL-11 could stimulate the transcriptional expression of the reporter gene and had a well-defined dose effect. The EC50 of IL-11 in stimulating the transcriptional expression of STAT3-luc reporter gene was 0.11 ng/ml.

**(2) Effect of hz8C8, hz18A10 and their mutants on STAT3 signaling pathway determined using the reporter gene system**

Activity of the antibodies for inhibiting the stimulation of the cell reporter gene by IL-11 was determined using the IL-11Rα/GP130/STAT3-luc HEK293 reporter gene system. The cells were inoculated in 96-well plates; and IL-11 was diluted to 0.3 ng/ml and co-incubated with an antibody gradiently diluted (10 μg/ml, and then 3-fold diluted to obtained 10 concentrations) for 30 minutes, then the mixtures were added to the cells for incubation for 6 hours. Later, the detection reagent in a Luciferase Assay kit (Vazyme Biotech Co., Ltd, D1201-02 bio-lite) was added, and signal values were read (RLUsample). In the present experiment about inhibitory activity, 3C6-hFc was used as a positive control antibody, an irrelevant human IgG (NC-hIgG) was used as a negative control antibody, PBS with IL-11 added was used as a positive blank control (RLUhigh), and PBS was used as a negative blank control (RLUlow).

inhibition % was calculated for each well using the readings from the wells having the antibody added at different concentrations, and the calculation formula of the inhibition % is as follows:

$$\text{inhibition \%} = (\text{RLUhigh-RLUsample}) / (\text{RLUhigh-RLUlow}) \times 100\%$$

A dose-effect curve was plotted through fitting to a four-parameter equation using GraphPad Prism software, by plotting the concentrations of the antibody on the X-axis against the averaged % inhibition on the Y-axis, and then IC50 value of each antibody was determined.

According to the method above, hz8C8, hz18A10 and their mutants were analyzed for their activity of inhibiting the transcriptional expression of the cell reporter gene stimulated by IL-11. The results (Table 8) showed that most of the mutants maintained blocking activity substantially identical to that of the parent antibodies.

Table 8. Results of blocking activity of hz8C8, hz18A10 and their mutants on the reporter gene system

| Sample information | | | Blocking activity (IC50, μg/ml) |
|---|---|---|---|
| Antibody | Heavy chain variable region | Light chain variable region | |
| hz8C8, parent | hz8C8-VH (SEQ ID NO. 11) | hz8C8-VL (SEQ ID NO.12) | 2.04 |
| hz8C8 mutants | hz8C8-VHm1 (SEQ ID NO.15) | hz8C8-VL (SEQ ID NO.12) | 2.13 |
| | hz8C8-VHm2 (SEQ ID NO.16) | hz8C8-VL (SEQ ID NO.12) | 0.51 |
| | hz8C8-VHm3 (SEQ ID NO.17) | hz8C8-VL (SEQ ID NO.12) | 0.946 |
| | hz8C8-VHm4 (SEQ ID NO.18) | hz8C8-VL (SEQ ID NO.12) | 1.542 |
| | hz8C8-VHm1 (SEQ ID NO.15) | hz8C8-VLm1 (SEQ ID NO.34) | 0.804 |
| | hz8C8-VHm1 (SEQ ID NO.15) | hz8C8-VLm2 (SEQ ID NO.35) | 0.038 |
| | hz8C8-VHm1 (SEQ ID NO.15) | hz8C8-VLm3 (SEQ ID NO.36) | 0.125 |
| | hz8C8-VHm1 (SEQ ID NO.15) | hz8C8-VLm4 (SEQ ID NO.37) | 0.098 |
| | hz8C8-VHm1 (SEQ ID NO.15) | hz8C8-VLm5 (SEQ ID NO.38) | 0.1 |
| | hz8C8-VHm1 (SEQ ID NO.15) | hz8C8-VLm6 (SEQ ID NO.39) | 0.076 |
| | hz8C8-VHm1 (SEQ ID NO.15) | hz8C8-VLm7 (SEQ ID NO.40) | 0.017 |
| | hz8C8-VHm1 (SEQ ID NO.15) | hz8C8-VLm8 (SEQ ID NO.41) | 0.01 |
| | hz8C8-VHm1 (SEQ ID NO.15) | hz8C8-VLm9 (SEQ ID NO.42) | 0.012 |
| | hz8C8-VHm5 (SEQ ID NO.19) | hz8C8-VLm7 (SEQ ID NO.40) | 0.01 |
| | hz8C8-VHm6 (SEQ ID NO.20) | hz8C8-VLm7 (SEQ ID NO.40) | 0.066 |
| | hz8C8-VHm1-m1 (SEQ ID NO.21) | hz8C8-VLm7 (SEQ ID NO.40) | 0.046 |
| | hz8C8-VHm1-m2 (SEQ ID NO.22) | hz8C8-VLm7 (SEQ ID NO.40) | 0.052 |
| | hz8C8-VHm1-m3 (SEQ ID NO.23) | hz8C8-VLm7 (SEQ ID NO.40) | 0.053 |
| | hz8C8-VHm1 (SEQ ID NO.15) | hz8C8-VLm7-m1 (SEQ ID NO.43) | 0.068 |
| | hz8C8-VHm1 (SEQ ID NO.15) | hz8C8-VLm7-m2 (SEQ ID NO.44) | 0.024 |
| | hz8C8-VHm1 (SEQ ID NO.15) | hz8C8-VLm7-m3 (SEQ ID NO.45) | 0.017 |
| | hz8C8-VHm1 (SEQ ID NO.15) | hz8C8-VLm7-m4 (SEQ ID NO.46) | 0.021 |
| | hz8C8-VHm1-m3 (SEQ ID NO.23) | hz8C8-VLm7-m1 (SEQ ID NO.43) | 0.010 |
| | hz8C8-VHm1-m3 (SEQ ID NO.23) | hz8C8-VLm7-m2 (SEQ ID NO.44) | 0.009 |
| | hz8C8-VHm1-m3 (SEQ ID NO.23) | hz8C8-VLm7-m3 (SEQ ID NO.45) | 0.022 |
| | hz8C8-VHm1-m3 (SEQ ID NO.23) | hz8C8-VLm7-m4 (SEQ ID NO.46) | 0.020 |
| | hz8C8-VHm1-m4 (SEQ ID NO.24) | hz8C8-VLm7-m2 (SEQ ID NO.44) | 0.004 |
| | hz8C8-VHm1-m5 (SEQ ID NO.25) | hz8C8-VLm7-m2 (SEQ ID NO.44) | 0.194 |
| | hz8C8-VHm1-m6 (SEQ ID NO.26) | hz8C8-VLm7-m2 (SEQ ID NO.44) | 0.034 |
| | hz8C8-VHm1-m7 (SEQ ID NO.27) | hz8C8-VLm7-m2 (SEQ ID NO.44) | 0.027 |
| | hz8C8-VHm1-m8 (SEQ ID NO.28) | hz8C8-VLm7-m2 (SEQ ID NO.44) | 0.003 |
| | hz8C8-VHm1-m9 (SEQ ID NO.29) | hz8C8-VLm7-m2 (SEQ ID NO.44) | 0.002 |
| | hz8C8-VHm1-m10 (SEQ ID NO.30) | hz8C8-VLm7-m2 (SEQ ID NO.44) | 0.008 |
| | hz8C8-VHm1-m11 (SEQ ID NO.31) | hz8C8-VLm7-m2 (SEQ ID NO.44) | 0.010 |
| | hz8C8-VHm1-m12 (SEQ ID NO.32) | hz8C8-VLm7-m2 (SEQ ID NO.44) | 0.012 |

(continued)

| Sample information | | | Blocking activity (IC50, μg/ml) |
|---|---|---|---|
| Antibody | Heavy chain variable region | Light chain variable region | |
| | hz8C8-VHm1-m13 (SEQ ID NO.33) | hz8C8-VLm7-m2 (SEQ ID NO.44) | 0.008 |
| hz18A10, parent | hz18A10-VH (SEQ ID NO.9) | hz18A10-VL (SEQ ID NO.10) | 18.54 |
| hz18A10 mutants | hz18A10-VH (SEQ ID NO.9) | hz18A10-VLm1 (SEQ ID NO.69) | 19.27 |
| | hz18A10-VH (SEQ ID NO.9) | hz18A10-VLm2 (SEQ ID NO.70) | 17.44 |
| | hz18A10-VH (SEQ ID NO.9) | hz18A10-VLm3 (SEQ ID NO.71) | 15.25 |
| | hz18A10-VH (SEQ ID NO.9) | hz18A10-VLm4 (SEQ ID NO.72) | 16.38 |
| | hz18A10-VH (SEQ ID NO.9) | hz18A10-VLm5 (SEQ ID NO.73) | 18.08 |
| | hz18A10-VH (SEQ ID NO.9) | hz18A10-VLm6 (SEQ ID NO.74) | 2.6 |
| | hz18A10-VH (SEQ ID NO.9) | hz18A10-VLm8 (SEQ ID NO.76) | 3.12 |
| | hz18A10-VH (SEQ ID NO.9) | hz18A10-VLm9 (SEQ ID NO.77) | 1.25 |
| | hz18A10-VH (SEQ ID NO.9) | hz18A10-VLm10 (SEQ ID NO.78) | 12.72 |
| | hz18A10-VH (SEQ ID NO.9) | hz18A10-VLm11 (SEQ ID NO.79) | 4.07 |
| | hz18A10-VH (SEQ ID NO.9) | hz18A10-VLm12 (SEQ ID NO.80) | 11.55 |
| | hz18A10-VH (SEQ ID NO.9) | hz18A10-VLm13 (SEQ ID NO.81) | 14.23 |
| | hz18A10-VHm1 (SEQ ID NO.49) | hz18A10-VL (SEQ ID NO.10) | 10.88 |
| | hz18A10-VHm2 (SEQ ID NO.50) | hz18A10-VL (SEQ ID NO.10) | 11.64 |
| | hz18A10-VHm1 (SEQ ID NO.49) | hz18A10-VLm7 (SEQ ID NO.75) | 13.07 |
| | hz18A10-VHm1 (SEQ ID NO.49) | hzl8A10-VLm14 (SEQ ID NO.82) | 9.68 |
| | hz18A10-VHm1 (SEQ ID NO.49) | hz18A10-VLm15 (SEQ ID NO.83) | 1.88 |
| | hz18A10-VHm1 (SEQ ID NO.49) | hz18A10-VLm9 (SEQ ID NO.77) | 11.12 |
| | hz18A10-VHm1 (SEQ ID NO.49) | hz18A10-VLm11 (SEQ ID NO.79) | 7.4 |
| | hz18A10-VHm2 (SEQ ID NO.50) | hz18A10-VLm9 (SEQ ID NO.77) | 13.86 |
| | hz18A10-VHm2 (SEQ ID NO.50) | hz18A10-VLm11 (SEQ ID NO.79) | 9.46 |
| | hz18A10-VHm5 (SEQ ID NO.53) | hz18A10-VLm9 (SEQ ID NO.77) | 11.59 |
| | hz18A10-VH (SEQ ID NO.9) | hz18A10-VLm16 (SEQ ID NO.84) | 9.89 |
| | hz18A10-VH (SEQ ID NO.9) | hz18A10-VLm17 (SEQ ID NO.85) | 3.47 |
| | hz18A10-VHm3 (SEQ ID NO.51) | hz18A10-VLm9 (SEQ ID NO.77) | 0.248 |
| | hz18A10-VHm4 (SEQ ID NO.52) | hz18A10-VLm9 (SEQ ID NO.77) | 2.23 |
| | hz18A10-VHm6 (SEQ ID NO.54) | hz18A10-VLm9 (SEQ ID NO.77) | 0.034 |
| | hz18A10-VH (SEQ ID NO.9) | hz18A10-VLm18 (SEQ ID NO.86) | 4.38 |
| | hz18A10-VHm4 (SEQ ID NO.52) | hz18A10-VLm18 (SEQ ID NO.86) | 1.94 |
| | hz18A10-VHm7 (SEQ ID NO.55) | hz18A10-VLm19 (SEQ ID NO.87) | 1.22 |
| | hz18A10-VHm8 (SEQ ID NO.56) | hz18A10-VLm19 (SEQ ID NO.87) | 8.64 |
| | hz18A10-VHm9 (SEQ ID NO.57) | hz18A10-VLm19 (SEQ ID NO.87) | 0.99 |
| | hz18A10-VHm3 (SEQ ID NO.51) | hz18A10-VLm19 (SEQ ID NO.87) | 0.262 |
| | hz18A10-VHm6 (SEQ ID NO.54) | hz18A10-VLm19 (SEQ ID NO.87) | 0.017 |
| | hz18A10-VHm10 (SEQ ID NO.58) | hz18A10-VLm19 (SEQ ID NO.87) | 0.136 |
| | hz18A10-VHm11 (SEQ ID NO.59) | hz18A10-VLm19 (SEQ ID NO.87) | 0.112 |

(continued)

| Sample information | | | Blocking activity (IC50, μg/ml) |
|---|---|---|---|
| **Antibody** | **Heavy chain variable region** | **Light chain variable region** | |
| | hz18A10-VHm12 (SEQ ID NO.60) | hz18A10-VLm19 (SEQ ID NO.87) | 0.011 |
| | hz18A10-VHm13 (SEQ ID NO.61) | hz18A10-VLm19 (SEQ ID NO.87) | 0.338 |
| | hz18A10-VHm14 (SEQ ID NO.62) | hz18A10-VLm19 (SEQ ID NO.87) | 1.03 |
| | hz18A10-VHm15 (SEQ ID NO.63) | hz18A10-VLm19 (SEQ ID NO.87) | 0.511 |
| | hz18A10-VHm16 (SEQ ID NO.64) | hz18A10-VLm19 (SEQ ID NO.87) | 0.034 |
| | hz18A10-VHm17 (SEQ ID NO.65) | hz18A10-VLm19 (SEQ ID NO.87) | 0.013 |
| | hz18A10-VHm18 (SEQ ID NO.66) | hz18A10-VLm19 (SEQ ID NO.87) | 0.79 |
| | hz18A10-VHm19 (SEQ ID NO.67) | hz18A10-VLm19 (SEQ ID NO.87) | 0.13 |
| | hz18A10-VHm20 (SEQ ID NO.68) | hz18A10-VLm19 (SEQ ID NO.87) | 0.061 |
| **Positive control** 3C6-hFc | 3C6-VH | 3 C6-VL | 0.342 |

## Example 8 Pharmacodynamic evaluation of anti-IL-11 antibodies in ConA-induced acute liver injury model

### (1) Establishment of ConA-induced acute liver injury model

A mouse model of acute liver injury was established with ConA induction, and the correlation between IL-11 and liver injury was analyzed.

12 Balb/c mice were randomly divided into 2 groups, 6 mice in each, i.e., a blank group and a model group. The mice in the model group were injected with Con A (Sigma) via tail veins to induce and establish an acute liver injury model. 24 hours after the modeling, blood was collected from infraorbital veins, sera were separated, and the activities of glutamic-pyruvic transaminase and glutamic-oxaloacetic transaminase were detected respectively, according to the instructions of a Glutamic-Pyruvic Transaminase Activity Assay kit and a Glutamic-Oxaloacetic Transaminase Activity Assay kit. The mice were euthanized after blood was collected, and liver left lobe tissues were taken. The obtained tissues were fixed with 10% formaldehyde, embedded in paraffin, sectioned, and HE stained for histopathological examination and pathological lesion scoring, and also for detection of IL-11 therein using an anti-mouse IL-11 antibody (Invitrogen) via IHC.

Following the protocol above, a mouse model of ConA-induced acute liver injury was established. The experiment results showed that the activities of glutamic-pyruvic transaminase and glutamic-oxaloacetic transaminase in the sera from the mice modeled with Con A remarkably increased (P < 0.01) (Table 9). The histopathological examination indicated that the established liver injury model was mainly characterized by inflammatory cell infiltration and hepatocyte necrosis. The infiltrated inflammatory cells were mainly located in the portal area, and then around the central vein and in the hepatic lobule, and were majorly neutrophilic granulocytes or lymphocytes, etc. The necrosis was coagulative necrosis, distributed as plaques, and necrotic lesions in severe areas interconnected and were bridge-shaped (Table 10). Those results demonstrate that Con A can cause acute liver injury in mice. The detection results from IHC staining showed that the expression of IL-11 significantly increased in the mouse model of ConA-induced acute liver injury, suggesting there is a correlation between acute liver injury and the increased IL-11 expression (Table 10).

Table 9. Serum biochemical indexes of mouse liver injury induced by Con A ($\bar{x}\pm$sd, n = 6)

| Group | Serum biochemical indexes | |
|---|---|---|
| | ALT(UAL) | AST(U/L) |
| Blank group | 8.03±3.94 | 10.50±4.76 |
| Model group | 34.63±8.09** | 36.72±12.82** |
| Compared with the blank group: ** *P* < 0.01. | | |

Table 10. Results of the scoring of mouse liver injury induced by Con A($\overline{x}\pm$sd, n = 6)

| Group | Lesion scoring | Gray value by IHC staining of IL-11 |
|---|---|---|
| Blank group | 0.00±0.00 | 1.060±0.064 |
| Model group | 4.917±1.625** | 7.213±0.347** |
| Compared with the blank group: ** *P* < 0.01, * *P* < 0.05 | | |

## (2) Pharmacodynamic evaluation of anti-IL-11 antibodies in ConA-induced acute liver injury model

70 Balb/c mice were randomly divided into 7 groups, 10 mice in each, i.e., a normal group, as well as six model groups, group G1, group G2, group G3, group G4, group G5, and group G6. Except for the mice in the normal group, the mice in other groups were injected with Con A at 10 mg/kg via tail veins. All the mice were fasted but had free access to water. No antibody was administered to the mice in the normal group; an isotype-IgG was administered to the mice in group G1 which was used as a model group; an antibody was administered to the mice in the group G2 1 hour before induced with Con A, which group was used as a prevention group; and an antibody was administered to the mice in all the other groups 2 hours after induced with Con A via tail veins (0.2 ml/20 g body weight, Table 11). After the administration, all the animals were fasted but had free access to water. 24 hours after the last administration, the mice were weighted and blood was sampled from the orbit. Sera were separated, and the activities of glutamic-pyruvic transaminase and glutamic-oxaloacetic transaminase were detected respectively, according to the instructions of a Glutamic-Pyruvic Transaminase Activity Assay kit and a Glutamic-Oxaloacetic Transaminase Activity Assay kit. Liver left lobe tissues were taken, and the obtained tissues were fixed with 10% formaldehyde, embedded in paraffin, sectioned, and HE stained for histopathological examination and pathological lesion scoring. All the data were processed, rank sum test was performed on data of the pathological lesion scoring, and t test was performed on other data, and the results were analyzed statistically.

Following the protocol above, the protective effect of anti-IL-11 antibodies against ConA-induced acute liver injury in the mouse model of ConA-induced acute liver injury was evaluated. The results are as follows.

As can be seen from Table 11, the activities of glutamic-pyruvic transaminase, glutamic-oxaloacetic transaminase, and tumor necrosis factor-$\alpha$ in the sera from the mice modeled with Con A remarkably increased (P < 0.01), indicating Con A can cause acute liver injury in mice. Compared with the model group, the administration in group G2, group G4, group G5 and group G6 significantly reduced the levels of glutamic-pyruvic transaminase, glutamic-oxalacetic transaminase, and tumor necrosis factor-$\alpha$ in the sera from the mice with acute and experimental lung injury, showing obvious statistical differences (P < 0.05 or P < 0.01); and the administration in group G3 reduced the levels of glutamic-pyruvic transaminase, glutamic-oxalacetic transaminase and tumor necrosis factor-alpha in the mice with acute and experimental lung injury.

The histopathological examination indicated (Table 12) that the established liver injury model was mainly characterized by inflammatory cell infiltration and hepatocyte necrosis. Inflammatory cells were mainly located in the portal area, and then around the central vein and in the hepatic lobule, and were majorly neutrophilic granulocytes or lymphocytes, etc. The necrosis was coagulative necrosis, distributed as plaques, and necrotic lesions in severe areas interconnected and were bridge-shaped. After the anti-IL-11 antibodies were administered, the degrees of inflammation and necrosis of liver tissues were obviously decreased, and the effects of the anti-IL-11 antibodies in sequence from high to low were: group G5, group G4, group G2, group G6, and group G3.

The above results indicate that anti-IL-11 antibodies hz8C8m7 (variable regions are hz8C8-VHm1+hz8C8-VLm7) and hz18A10m19 (variable regions are hz18A10-VHm19+hz18A10-VLm19) have a preventive or protective effect against ConA-induced acute liver injury in mice.

Table 11. Results of the effect of anti-IL-11 antibodies on the serum biochemical indexes of mouse liver injury induced by Con A

| Group | | Serum biochemical indexes | | Cytokine |
|---|---|---|---|---|
| | | ALT (U/L) | AST (U/L) | TNF-$\alpha$ (ng/L) |
| Normal group | / | 6.66±2.72** | 7.86±3.54** | 62.36±18.98** |
| Group G1 | Isotype-IgG | 40.66±9.58 | 37.58±12.46 | 112.51±27.99 |
| Group G2 | hz8C8m7 0.8 mg/ml | 21.20±11.49* | 12.86±6.68** | 75.32±21.58** |
| Group G3 | hz8C8m7 0.2 mg/ml | 33.42±7.09 | 28.76±9.96 | 89.75±32.39 |
| Group G4 | hz8C8m7 0.8 mg/ml | 17.80±6.38** | 11.37±7.95** | 78.03±18.71** |

(continued)

| Group | | Serum biochemical indexes | | Cytokine |
| --- | --- | --- | --- | --- |
| | | ALT (U/L) | AST (U/L) | TNF-$\alpha$ (ng/L) |
| Group G5 | hz8C8m7 2.4 mg/ml | 20.56±7.88** | 9.39±5.69** | 60.52±16.74** |
| Group G6 | hz18A10m19 0.8 mg/ml | 26.15±7.59** | 17.46±7.73** | 75.07±30.25* |
| Compared with the model group: ** $P$ < 0.01, * $P$ < 0.05. | | | | |

Table 12. Results of the effect of anti-IL-11 antibodies on the scoring of mouse liver injury induced by Con A ( $\bar{x}$±sd, n = 10)

| Group | | lesion scoring |
| --- | --- | --- |
| Normal group | / | 0.00±0.00** |
| Group G1 | Isotype-IgG | 5.00±2.21 |
| Group G2 | hz8C8m7 0.8 mg/ml | 2.55±0.86** |
| Group G3 | hz8C8m7 0.2 mg/ml | 3.25±2.31 |
| Group G4 | hz8C8m7 0.8 mg/ml | 2.30±1.70** |
| Group G5 | hz8C8m7 2.4 mg/ml | 2.30±1.34** |
| Group G6 | hz18A10m19 0.8 mg/ml | 2.55±0.98** |
| Compared with the model group: ** $P$ < 0.01. | | |

## Example 9 Pharmacodynamic evaluation of anti-IL-11 antibodies in BLM-induced pulmonary fibrosis model

Activity of pharmacodynamics of the anti-IL-11 antibodies was evaluated in BLM-induced pulmonary fibrosis model. Mice were randomly divided into 6 groups, in which the blank control group had 5 mice to which physiological saline was administered via tracheas, and the other 5 groups had 10 mice in each to which bleomycin (2 U/kg) was administered with via tracheas to establish pulmonary fibrosis model. The 5 groups with bleomycin administered are as follows:

Bleomycin model group (BLM): saline was administered;
Positive drug Nintedanib group (Nintedanib): Nintedanib was administered at 100 mg/kg;
3C6-hFc group (hz3C6): 3C6-hFc (variable regions are 3C6-VH and 3C6-VL, and constant regions are as shown in SEQ ID NO.13 and SEQ ID NO.14) was administered at 20 mg/kg;
hz8C8m7 group: hz8C8m7 (variable regions are hz8C8-VHm1 and hz8C8-VLm7, and constant regions are as shown in SEQ ID NO.13 and SEQ ID NO.14) was administered at 20 mg/kg;
hz18A10m19 group: hz18A10m19 (variable regions are hz18A10-VHm19 and hz18A10-VLm19, and constant regions are as shown in SEQ ID NO.13 and SEQ ID NO.14) was administered at 20 mg/kg.

Starting on the 2nd day after the modeling, the antibodies were injected intraperitoneally once every two days, for 8 times. The weight of the mice were continuously detected in the administration process; and on the 18th day after the modeling, the lung function of the mice was determined, and lung tissues were sampled for the detection of pathological indexes, including H&E and Masson staining of the lung tissue sections, and analyzing of the areas of lung fibrosis.

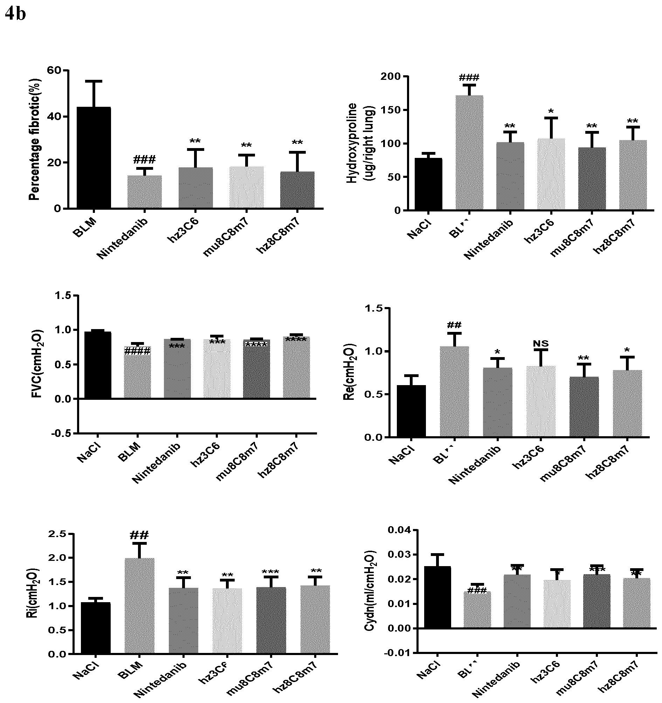

Experiment results showed (FIG. 4) that no change in fibrosis related indexes and impaired lung function were observed in the blank control group to which physiological saline was administered. In the bleomycin model group, obvious pulmonary fibrosis was seen and hydroxyproline (HYP) in the lung tissues obviously increased, and the lung function was seriously damaged. Different degrees of protection effects were shown in the treatment groups administered with 20 mg/kg of antibodies 3C6-hFc, hz8C8m7, and hz18A10m19, reflected by the reduction of fibrosis related indexes and the improvement of lung function.

## Example 10 Pharmacodynamic evaluation of anti-IL-11 antibodies in a mouse tumor model

Six weeks old female C57 BL/6 mice were subcutaneously inoculated with $3\times10^6$ murine MC38 colon cancer cells and randomly grouped with 6 mice per group when tumors grew to around 70 mm$^3$. Grouping as well as administration dosage and frequency for each group are shown in Table 13. Each group was injected intravenously twice a week for 3 times; at the same time, tumor volume and body weight of each mouse were measured. When a mouse lost weight more than 15%, or when an animal had a tumor volume exceeding 3000 mm$^3$, or when a whole group of animals had an average tumor volume exceeding 2000 mm$^3$, the experiment was stopped, and the mouse/mice was/were euthanized.

Table 13. Grouping as well as administration dosage and frequency for the C57BL/6 mice bearing MC38 tumor

| Group (n = 6) | Drug | Administration dosage (mg/kg) | Administration frequency |
|---|---|---|---|
| 1 | hz8C8m7 | 20 | Biw×3 |
| 2 | anti-mPD1 Ab* | 5 | Biw×3 |
| 3 | hz8C8m7 + anti-mPD1 Ab | 20+5 | Biw×3 |
| 4 | NC-mIgG1 | 20 | Biw×3 |

*: The anti-mPD1 Ab is an antibody capable of binding to murine PD-1 (see the patent publication WO2019062755A1; "WBP336C"), and the sequences of the heavy chain variable region (WBP336C-VH) and the light chain variable region (WBP336C-VL) of the antibody were shown below; and the sequences of the heavy chain constant region and the light chain constant region are shown in SEQ ID NO.3 and SEQ ID NO.4 respectively.

WBP336C-VH (SEQ ID NO.47)

QVQLVQSGAEVKKPGSSVKVSCKASGFTFTTYYISWVRQAPGQGLEYLGYINMGSGG TNYNEKFKGRVTITADKSTSTAYMELSSLRSEDTAVYYCAIIGYFDYWGQGTMVTVSS

WBP336C-VL (SEQ ID NO.48)

DVVMTQSPLSLPVTLGQPASISCRSSQSLLDSDGGTYLYWFQQRPGQSPRRLIYLVSTL GSGVPDRFSGSGSGTDFTLKISRVEAEDVGVYYCMQLTHWPYTFGQGTKLEIK

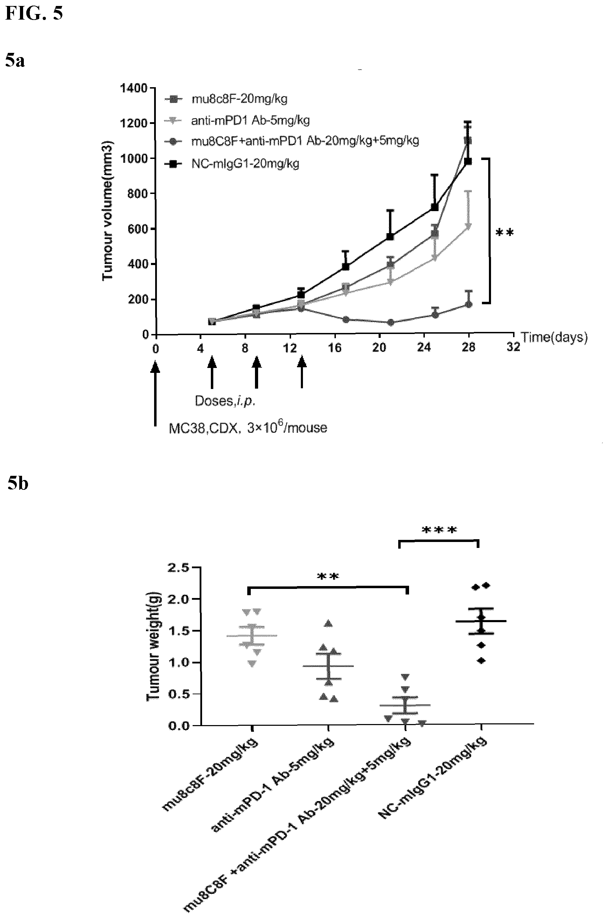

As can be seen from FIG. 5, in the C57BL/6 mouse model of MC38 tumor subcutaneous xenograft, the combination use of hz8C8m7 and anti-mPD1 Ab obviously inhibited the tumor growth, as compared with the mice in the control group administered with the isotype antibody NC-mIgG1, and the difference was statistically significant. The results suggested that hz8C8m7 could enhance the therapeutic effect of the anti-PD-1 Ab, indicating a synergistic anti-tumor effect of hz8C8m7 and a therapeutic antibody against PD-1/PD-L1 pathway.

The above description of the embodiments of the present disclosure is not intended to limit the present disclosure, and those skilled in the art may make various changes and modifications to the present disclosure without departing from the spirit of the present disclosure, which should fall within the scope of the appended claims.

**Claims**

1. An antibody or fragment thereof comprising a heavy chain variable region (VH) and a light chain variable region (VL) which comprise a combination of heavy chain CDRs and light chain CDRs (H-CDR1, H-CDR2, and H-CDR3; and, L-CDR1, L-CDR2, and L-CDR3) selected from the group consisting of:

(1)

(1-1) H-CDR1, H-CDR2, and H-CDR3 comprising the amino acid sequences as shown in SEQ ID NO.94, SEQ ID NO.95, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-2) H-CDR1, H-CDR2, and H-CDR3 comprising the amino acid sequences as shown in SEQ ID NO.94, SEQ ID NO.100, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-3) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-4) H-CDR1, H-CDR2, and H-CDR3 comprising the amino acid sequences as shown in SEQ ID NO.94, SEQ ID NO.102, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-5) H-CDR1, H-CDR2, and H-CDR3 comprising the amino acid sequences as shown in SEQ ID NO.94, SEQ ID NO.103, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-6) H-CDR1, H-CDR2, and H-CDR3 comprising the amino acid sequences as shown in SEQ ID NO.94, SEQ ID NO.104, and SEQ ID NO.96 successively; and, L-CDR1, L-CDR2, and L-CDR3 comprising the amino acid sequences as shown in SEQ ID NO.97, SEQ ID NO.98, and SEQ ID NO.99 successively;

(1-7) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 115, SEQ ID NO.98, and SEQ ID NO.99 successively;

(1-8) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.116, SEQ ID NO.98, and SEQ ID NO.99 successively;

(1-9) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.117, SEQ ID NO.98, SEQ ID NO.99 successively;

(1-10) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 118, SEQ ID NO.98, and SEQ ID NO.99 successively;

(1-11) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 119, SEQ ID NO.98, and SEQ ID NO.99 successively;

(1-12) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 120, SEQ ID NO.98, and SEQ ID NO.99 successively;

(1-13) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-14) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 122, SEQ ID NO.98, and SEQ ID NO.99 successively;

(1-15) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO. 123, SEQ ID NO.98, and SEQ ID NO.99 successively;

(1-16) H-CDR1, H-CDR2, and H-CDR3 comprising the amino acid sequences as shown in SEQ ID NO.105, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-17) H-CDR1, H-CDR2, and H-CDR3 comprising the amino acid sequences as shown in SEQ ID NO.94, SEQ ID NO.106, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-18) H-CDR1, H-CDR2, and H-CDR3 comprising the amino acid sequences as shown in SEQ ID NO.107, SEQ ID NO.101, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-19) H-CDR1, H-CDR2, and H-CDR3 comprising the amino acid sequences as shown in SEQ ID NO.94, SEQ ID NO.108, and SEQ ID NO.96 successively; and, L-CDR1, L-CDR2, and L-CDR3 comprising the amino acid sequences as shown in SEQ ID NO. 121, SEQ ID NO.98, and SEQ ID NO.99 successively;

(1-20) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO. 121, SEQ ID NO.98, and SEQ ID NO.99 successively;

(1-21) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.124, and SEQ ID NO.99 successively;

(1-22) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.125, and SEQ ID NO.99 successively;

(1-23) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.126, and SEQ ID NO.99 successively;

(1-24) H-CDR1, H-CDR2, and H-CDR3 comprising the amino acid sequences as shown in SEQ ID NO.94, SEQ ID NO.101, and SEQ ID NO.96 successively; and, L-CDR1, L-CDR2, and L-CDR3 comprising the amino acid sequences as shown in SEQ ID NO.121, SEQ ID NO.127, and SEQ ID NO.99 successively;

(1-25) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.124, and SEQ ID NO.99 successively;

(1-26) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.125, and SEQ ID NO.99 successively;

(1-27) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.126, and SEQ ID NO.99 successively;

(1-28) H-CDR1, H-CDR2, and H-CDR3 comprising the amino acid sequences as shown in SEQ ID NO.94, SEQ ID NO.101, and SEQ ID NO.109 successively; and, L-CDR1, L-CDR2, and L-CDR3 comprising the amino acid sequences as shown in SEQ ID NO.121, SEQ ID NO.127, and SEQ ID NO.99 successively;

(1-29) H-CDR1, H-CDR2, and H-CDR3 comprising the amino acid sequences as shown in SEQ ID NO.94, SEQ ID NO.110, and SEQ ID NO.96 successively; and, L-CDR1, L-CDR2, and L-CDR3 comprising the amino acid sequences as shown in SEQ ID NO.121, SEQ ID NO.125, and SEQ ID NO.99 successively;

(1-30) H-CDR1, H-CDR2, and H-CDR3 comprising the amino acid sequences as shown in SEQ ID NO.94, SEQ ID NO.101, and SEQ ID NO.111 successively; and, L-CDR1, L-CDR2, and L-CDR3 comprising the amino acid sequences as shown in SEQ ID NO.121, SEQ ID NO. 125, and SEQ ID NO.99 successively;

(1-31) H-CDR1, H-CDR2, and H-CDR3 comprising the amino acid sequences as shown in SEQ ID NO.94, SEQ ID NO.101, and SEQ ID NO.112 successively; and, L-CDR1, L-CDR2, and L-CDR3 comprising the amino acid sequences as shown in SEQ ID NO.121, SEQ ID NO.125, and SEQ ID NO.99 successively;

(1-32) H-CDR1, H-CDR2, and H-CDR3 comprising the amino acid sequences as shown in SEQ ID NO.94, SEQ ID NO.101, and SEQ ID NO.113 successively; and, L-CDR1, L-CDR2, and L-CDR3 comprising the amino acid sequences as shown in SEQ ID NO.121, SEQ ID NO.125, and SEQ ID NO.99 successively; and

(1-33) H-CDR1, H-CDR2, and H-CDR3 comprising the amino acid sequences as shown in SEQ ID NO.94, SEQ ID NO.101, and SEQ ID NO.114 successively; and, L-CDR1, L-CDR2, and L-CDR3 comprising the amino acid sequences as shown in SEQ ID NO.121, SEQ ID NO.125, and SEQ ID NO.99 successively; alternatively,

(2)

(2-1) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.92, and SEQ ID NO.93 successively;

(2-2) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.146, SEQ ID NO.92, and SEQ ID NO.93 successively;

(2-3) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.147, SEQ ID NO.92, and SEQ ID NO.93 successively;

(2-4) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.148, SEQ ID NO.92, and SEQ ID NO.93 successively;

(2-5) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.149, SEQ ID NO.92, and SEQ ID NO.93 successively;

(2-6) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.150, SEQ ID NO.92, and SEQ ID NO.93 successively;

(2-7) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.151, SEQ ID NO.92, and SEQ ID NO.93 successively;

(2-8) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.153, SEQ ID NO.92, and SEQ ID NO.93 successively;

(2-9) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.154, SEQ ID NO.92, and SEQ ID NO.93 successively;

(2-10) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO. 155, SEQ ID NO.92, and SEQ ID NO.93 successively;

(2-11) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.156, and SEQ ID NO.93 successively;

(2-12) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.157, and SEQ ID NO.93 successively;

(2-13) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.158, and SEQ ID NO.93 successively;

(2-14) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO.92, and SEQ ID NO.93 successively;

(2-15) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO.91, SEQ ID NO.92, and SEQ ID NO.93 successively;

(2-16) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO. 152, SEQ ID NO.92, and SEQ ID NO.93 successively;

(2-17) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO.159, and SEQ ID NO.93 successively;

(2-18) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO.160, and SEQ ID NO.93 successively;

(2-19) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO. 154, SEQ ID NO.92, and SEQ ID NO.93 successively;

(2-20) H-CDR1, H-CDR2, and H-CDR3 comprising the amino acid sequences as shown in SEQ ID NO.88, SEQ ID NO.89, and SEQ ID NO.130 successively; and, L-CDR1, L-CDR2, and L-CDR3 comprising the amino acid sequences as shown in SEQ ID NO.91, SEQ ID NO.156, and SEQ ID NO.93 successively;

(2-21) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO. 154, SEQ ID NO.92, and SEQ ID NO.93 successively;

(2-22) H-CDR1, H-CDR2, and H-CDR3 comprising the amino acid sequences as shown in SEQ ID NO.88, SEQ ID NO.89, and SEQ ID NO.131 successively; and, L-CDR1, L-CDR2, and L-CDR3 comprising the amino acid sequences as shown in SEQ ID NO.91, SEQ ID NO.156, and SEQ ID NO.93 successively;

(2-23) H-CDR1, H-CDR2, and H-CDR3 comprising the amino acid sequence shown in sequence in SEQ ID NO.88, SEQ ID NO.89, and SEQ ID NO.134 successively; and, L-CDR1, L-CDR2, and L-CDR3 comprising the amino acid sequences as shown in SEQ ID NO.154, SEQ ID NO.92, and SEQ ID NO.93 successively;

(2-24) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.91, SEQ ID NO.161, and SEQ ID NO.93 successively;

(2-25) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.162, SEQ ID NO.156, and SEQ ID NO.93 successively;

(2-26) H-CDR1, H-CDR2, and H-CDR3 comprising the amino acid sequences as shown in SEQ ID NO.132, SEQ ID NO.89, and SEQ ID NO.90 successively; and, L-CDR1, L-CDR2, and L-CDR3 comprising the amino acid sequences as shown in SEQ ID NO. 154, SEQ ID NO.92, and SEQ ID NO.93 successively;

(2-27) H-CDR1, H-CDR2, and H-CDR3 comprising the amino acid sequences as shown in SEQ ID NO.133, SEQ ID NO.89, SEQ ID NO.90 successively; and, L-CDR1, L-CDR2, and L-CDR3 comprising the amino acid sequences as shown in SEQ ID NO.154, SEQ ID NO.92, and SEQ ID NO.93 successively;

(2-28) H-CDR1, H-CDR2, and H-CDR3 comprising the amino acid sequences as shown in SEQ ID NO.135, SEQ ID NO.89, and SEQ ID NO.90 successively; and, L-CDR1, L-CDR2, and L-CDR3 comprising the amino acid sequences as shown in SEQ ID NO. 154, SEQ ID NO.92, and SEQ ID NO.93 successively;

(2-29) H-CDR1, H-CDR2, and H-CDR3 comprising the amino acid sequences as shown in SEQ ID NO.88, SEQ ID NO.89, and SEQ ID NO.90 successively; and, L-CDR1, L-CDR2, and L-CDR3 comprising the amino acid sequences as shown in SEQ ID NO.162, SEQ ID NO.160, and SEQ ID NO.93 successively;

(2-30) H-CDR1, H-CDR2, and H-CDR3 comprising the amino acid sequences as shown in SEQ ID NO.133, SEQ ID NO.89, and SEQ ID NO.90 successively; and, L-CDR1, L-CDR2, and L-CDR3 comprising the amino acid sequences as shown in SEQ ID NO.162, SEQ ID NO.160, and SEQ ID NO.93 successively;

(2-31) H-CDR1, H-CDR2, and H-CDR3 comprising the amino acid sequences as shown in SEQ ID NO.136, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-32) H-CDR1, H-CDR2, and H-CDR3 comprising the amino acid sequences as shown in SEQ ID NO.137, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-33) H-CDR1, H-CDR2, and H-CDR3 comprising the amino acid sequences as shown in SEQ ID NO.138, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-34) H-CDR1, H-CDR2, and H-CDR3 comprising the amino acid sequences as shown in SEQ ID NO.132, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-35) H-CDR1, H-CDR2, and H-CDR3 comprising the amino acid sequences as shown in SEQ ID NO.135, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-36) H-CDR1, H-CDR2, and H-CDR3 comprising the amino acid sequences as shown in SEQ ID NO.139, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-37) H-CDR1, H-CDR2, and H-CDR3 comprising the amino acid sequences as shown in SEQ ID NO.140, SEQ ID NO.89, and SEQ ID NO.90 successively; and, L-CDR1, L-CDR2, and L-CDR3 comprising the amino acid sequences as shown in SEQ ID NO.162, SEQ ID NO.92, and SEQ ID NO.93 successively;

(2-38) H-CDR1, H-CDR2, and H-CDR3 comprising the amino acid sequences as shown in SEQ ID NO.141, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-39) H-CDR1, H-CDR2, and H-CDR3 comprising the amino acid sequences as shown in SEQ ID NO.142, SEQ ID NO.89, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-40) H-CDR1, H-CDR2, and H-CDR3 comprising the amino acid sequences as shown in SEQ ID NO.135, SEQ ID NO.143, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-41) H-CDR1, H-CDR2, and H-CDR3 comprising the amino acid sequences as shown in SEQ ID NO.135, SEQ ID NO.144, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-42) H-CDR1, H-CDR2, and H-CDR3 comprising the amino acid sequences as shown in SEQ ID NO.139, SEQ ID NO.144, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively;

(2-43) H-CDR1, H-CDR2, and H-CDR3 comprising the amino acid sequences as shown in SEQ ID NO.139, SEQ ID NO. 143, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively; and

(2-44) H-CDR1, H-CDR2, and H-CDR3 comprising the amino acid sequences as shown in SEQ ID NO.139, SEQ ID NO. 145, and SEQ ID NO.90 successively; and, L-CDR1, L-CDR2, and L-CDR3 comprising the amino acid sequences as shown in SEQ ID NO. 162, SEQ ID NO.92, and SEQ ID NO.93 successively.

2. The antibody or fragment thereof according to claim 1, **characterized in that** the antibody or fragment thereof is an anti-interleukin-11 antibody or fragment thereof; preferably, the IL-11 is human IL-11;

preferably, the anti-IL-11 antibody or fragment thereof comprises at least a heavy chain variable region and a light chain variable region, both of the heavy chain variable region and the light chain variable region comprising the combination of CDRs as defined in claim 1 and interspersed Framework Regions (FRs) respectively, which regions are arranged as follows: FR1-CDR1-FR2-CDR2-FR3 -CDR3 -FR4.

3.  The antibody or fragment thereof according to claim 1 or 2, **characterized in that** the heavy chain variable region and the light chain variable region comprise a combination of amino acid sequences selected from the group consisting of:

    (1)

    (1-1) the amino acid sequence as shown in SEQ ID NO.7, or an amino acid sequence having at least 75% identity to the amino acid sequence; and, the amino acid sequence as shown in SEQ ID NO.8, or an amino acid sequence having at least 75% identity to the amino acid sequence;
    (1-2) the amino acid sequence as shown in SEQ ID NO. 11, or an amino acid sequence having at least 75% identity to the amino acid sequence; and, the amino acid sequence as shown in SEQ ID NO.12, or an amino acid sequence having at least 75% identity to the amino acid sequence;
    (1-3) the amino acid sequence as shown in SEQ ID NO.15, or an amino acid sequence having at least 75% identity to the amino acid sequence; and, the amino acid sequence as shown in SEQ ID NO.12, or an amino acid sequence having at least 75% identity to the amino acid sequence;
    (1-4) the amino acid sequence as shown in SEQ ID NO.16, or an amino acid sequence having at least 75% identity to the amino acid sequence; and, the amino acid sequence as shown in SEQ ID NO.12, or an amino acid sequence having at least 75% identity to the amino acid sequence;
    (1-5) the amino acid sequence as shown in SEQ ID NO.17, or an amino acid sequence having at least 75% identity to the amino acid sequence; and, the amino acid sequence as shown in SEQ ID NO.12, or an amino acid sequence having at least 75% identity to the amino acid sequence;
    (1-6) the amino acid sequence as shown in SEQ ID NO.18, or an amino acid sequence having at least 75% identity to the amino acid sequence; and, the amino acid sequence as shown in SEQ ID NO.12, or an amino acid sequence having at least 75% identity to the amino acid sequence;
    (1-7) the amino acid sequence as shown in SEQ ID NO.15, or an amino acid sequence having at least 75% identity to the amino acid sequence; and, the amino acid sequence as shown in SEQ ID NO.34, or an amino acid sequence having at least 75% identity to the amino acid sequence;
    (1-8) the amino acid sequence as shown in SEQ ID NO.15, or an amino acid sequence having at least 75% identity to the amino acid sequence; and, the amino acid sequence as shown in SEQ ID NO.35, or an amino acid sequence having at least 75% identity to the amino acid sequence;
    (1-9) the amino acid sequence as shown in SEQ ID NO.15, or an amino acid sequence having at least 75% identity to the amino acid sequence; and, the amino acid sequence as shown in SEQ ID NO.36, or an amino acid sequence having at least 75% identity to the amino acid sequence;
    (1-10) the amino acid sequence as shown in SEQ ID NO.15, or an amino acid sequence having at least 75% identity to the amino acid sequence; and, the amino acid sequence as shown in SEQ ID NO.37, or an amino acid sequence having at least 75% identity to the amino acid sequence;
    (1-11) the amino acid sequence as shown in SEQ ID NO.15, or an amino acid sequence having at least 75% identity to the amino acid sequence; and, the amino acid sequence as shown in SEQ ID NO.38, or an amino acid sequence having at least 75% identity to the amino acid sequence;
    (1-12) the amino acid sequence as shown in SEQ ID NO.15, or an amino acid sequence having at least 75% identity to the amino acid sequence; and, the amino acid sequence as shown in SEQ ID NO.39, or an amino acid sequence having at least 75% identity to the amino acid sequence;
    (1-13) the amino acid sequence as shown in SEQ ID NO.15, or an amino acid sequence having at least 75% identity to the amino acid sequence; and, the amino acid sequence as shown in SEQ ID NO.40, or an amino acid sequence having at least 75% identity to the amino acid sequence;
    (1-14) the amino acid sequence as shown in SEQ ID NO.15, or an amino acid sequence having at least 75% identity to the amino acid sequence; and, the amino acid sequence as shown in SEQ ID NO.41, or an amino acid sequence having at least 75% identity to the amino acid sequence;
    (1-15) the amino acid sequence as shown in SEQ ID NO.15, or an amino acid sequence having at least 75% identity to the amino acid sequence; and, the amino acid sequence as shown in SEQ ID NO.42, or an amino acid sequence having at least 75% identity to the amino acid sequence;
    (1-16) the amino acid sequence as shown in SEQ ID NO.19, or an amino acid sequence having at least 75% identity to the amino acid sequence; and, the amino acid sequence as shown in SEQ ID NO.40, or an amino acid sequence having at least 75% identity to the amino acid sequence;
    (1-17) the amino acid sequence as shown in SEQ ID NO.20, or an amino acid sequence having at least 75% identity to the amino acid sequence; and, the amino acid sequence as shown in SEQ ID NO.40, or an amino acid sequence having at least 75% identity to the amino acid sequence;

(1-18) the amino acid sequence as shown in SEQ ID NO.21, or an amino acid sequence having at least 75% identity to the amino acid sequence; and, the amino acid sequence as shown in SEQ ID NO.40, or an amino acid sequence having at least 75% identity to the amino acid sequence;

(1-19) the amino acid sequence as shown in SEQ ID NO.22, or an amino acid sequence having at least 75% identity to the amino acid sequence; and, the amino acid sequence as shown in SEQ ID NO.40, or an amino acid sequence having at least 75% identity to the amino acid sequence;

(1-20) the amino acid sequence as shown in SEQ ID NO.23, or an amino acid sequence having at least 75% identity to the amino acid sequence; and, the amino acid sequence as shown in SEQ ID NO.40, or an amino acid sequence having at least 75% identity to the amino acid sequence;

(1-21) the amino acid sequence as shown in SEQ ID NO.15, or an amino acid sequence having at least 75% identity to the amino acid sequence; and, the amino acid sequence as shown in SEQ ID NO.43, or an amino acid sequence having at least 75% identity to the amino acid sequence;

(1-22) the amino acid sequence as shown in SEQ ID NO.15, or an amino acid sequence having at least 75% identity to the amino acid sequence; and, the amino acid sequence as shown in SEQ ID NO.44, or an amino acid sequence having at least 75% identity to the amino acid sequence;

(1-23) the amino acid sequence as shown in SEQ ID NO.15, or an amino acid sequence having at least 75% identity to the amino acid sequence; and, the amino acid sequence as shown in SEQ ID NO.45, or an amino acid sequence having at least 75% identity to the amino acid sequence;

(1-24) the amino acid sequence as shown in SEQ ID NO.15, or an amino acid sequence having at least 75% identity to the amino acid sequence; and, the amino acid sequence as shown in SEQ ID NO.46, or an amino acid sequence having at least 75% identity to the amino acid sequence;

(1-25) the amino acid sequence as shown in SEQ ID NO.23, or an amino acid sequence having at least 75% identity to the amino acid sequence; and, the amino acid sequence as shown in SEQ ID NO.43, or an amino acid sequence having at least 75% identity to the amino acid sequence;

(1-26) the amino acid sequence as shown in SEQ ID NO.23, or an amino acid sequence having at least 75% identity to the amino acid sequence; and, the amino acid sequence as shown in SEQ ID NO.44, or an amino acid sequence having at least 75% identity to the amino acid sequence;

(1-27) the amino acid sequence as shown in SEQ ID NO.23, or an amino acid sequence having at least 75% identity to the amino acid sequence; and, the amino acid sequence as shown in SEQ ID NO.45, or an amino acid sequence having at least 75% identity to the amino acid sequence;

(1-28) the amino acid sequence as shown in SEQ ID NO.23, or an amino acid sequence having at least 75% identity to the amino acid sequence; and, the amino acid sequence as shown in SEQ ID NO.46, or an amino acid sequence having at least 75% identity to the amino acid sequence;

(1-29) the amino acid sequence as shown in SEQ ID NO.24, or an amino acid sequence having at least 75% identity to the amino acid sequence; and, the amino acid sequence as shown in SEQ ID NO.44, or an amino acid sequence having at least 75% identity to the amino acid sequence;

(1-30) the amino acid sequence as shown in SEQ ID NO.25, or an amino acid sequence having at least 75% identity to the amino acid sequence; and, the amino acid sequence as shown in SEQ ID NO.44, or an amino acid sequence having at least 75% identity to the amino acid sequence;

(1-31) the amino acid sequence as shown in SEQ ID NO.26, or an amino acid sequence having at least 75% identity to the amino acid sequence; and, the amino acid sequence as shown in SEQ ID NO.44, or an amino acid sequence having at least 75% identity to the amino acid sequence;

(1-32) the amino acid sequence as shown in SEQ ID NO.27, or an amino acid sequence having at least 75% identity to the amino acid sequence; and, the amino acid sequence as shown in SEQ ID NO.44, or an amino acid sequence having at least 75% identity to the amino acid sequence;

(1-33) the amino acid sequence as shown in SEQ ID NO.28, or an amino acid sequence having at least 75% identity to the amino acid sequence; and, the amino acid sequence as shown in SEQ ID NO.44, or an amino acid sequence having at least 75% identity to the amino acid sequence;

(1-34) the amino acid sequence as shown in SEQ ID NO.29, or an amino acid sequence having at least 75% identity to the amino acid sequence; and, the amino acid sequence as shown in SEQ ID NO.44, or an amino acid sequence having at least 75% identity to the amino acid sequence;

(1-35) the amino acid sequence as shown in SEQ ID NO.30, or an amino acid sequence having at least 75% identity to the amino acid sequence; and, the amino acid sequence as shown in SEQ ID NO.44, or an amino acid sequence having at least 75% identity to the amino acid sequence;

(1-36) the amino acid sequence as shown in SEQ ID NO.31, or an amino acid sequence having at least 75% identity to the amino acid sequence; and, the amino acid sequence as shown in SEQ ID NO.44, or an amino acid sequence having at least 75% identity to the amino acid sequence;

(1-37) the amino acid sequence as shown in SEQ ID NO.32, or an amino acid sequence having at least 75% identity to the amino acid sequence; and, the amino acid sequence as shown in SEQ ID NO.44, or an amino acid sequence having at least 75% identity to the amino acid sequence; and

(1-38) the amino acid sequence as shown in SEQ ID NO.33, or an amino acid sequence having at least 75% identity to the amino acid sequence; and, the amino acid sequence as shown in SEQ ID NO.44, or an amino acid sequence having at least 75% identity to the amino acid sequence;

alternatively,

(2)

(2-1) the amino acid sequence as shown in SEQ ID NO.5, or an amino acid sequence having at least 75% identity to the amino acid sequence; and, the amino acid sequence as shown in SEQ ID NO.6, or an amino acid sequence having at least 75% identity to the amino acid sequence;

(2-2) the amino acid sequence as shown in SEQ ID NO.9, or an amino acid sequence having at least 75% identity to the amino acid sequence; and, the amino acid sequence as shown in SEQ ID NO.10, or an amino acid sequence having at least 75% identity to the amino acid sequence;

(2-3) the amino acid sequence as shown in SEQ ID NO.9, or an amino acid sequence having at least 75% identity to the amino acid sequence; and, the amino acid sequence as shown in SEQ ID NO.69, or an amino acid sequence having at least 75% identity to the amino acid sequence;

(2-4) the amino acid sequence as shown in SEQ ID NO.9, or an amino acid sequence having at least 75% identity to the amino acid sequence; and, the amino acid sequence as shown in SEQ ID NO.70, or an amino acid sequence having at least 75% identity to the amino acid sequence;

(2-5) the amino acid sequence as shown in SEQ ID NO.9, or an amino acid sequence having at least 75% identity to the amino acid sequence; and, the amino acid sequence as shown in SEQ ID NO.71, or an amino acid sequence having at least 75% identity to the amino acid sequence;

(2-6) the amino acid sequence as shown in SEQ ID NO.9, or an amino acid sequence having at least 75% identity to the amino acid sequence; and, the amino acid sequence as shown in SEQ ID NO.72, or an amino acid sequence having at least 75% identity to the amino acid sequence;

(2-7) the amino acid sequence as shown in SEQ ID NO.9, or an amino acid sequence having at least 75% identity to the amino acid sequence; and, the amino acid sequence as shown in SEQ ID NO.73, or an amino acid sequence having at least 75% identity to the amino acid sequence;

(2-8) the amino acid sequence as shown in SEQ ID NO.9, or an amino acid sequence having at least 75% identity to the amino acid sequence; and, the amino acid sequence as shown in SEQ ID NO.74, or an amino acid sequence having at least 75% identity to the amino acid sequence;

(2-9) the amino acid sequence as shown in SEQ ID NO.9, or an amino acid sequence having at least 75% identity to the amino acid sequence; and, the amino acid sequence as shown in SEQ ID NO.76, or an amino acid sequence having at least 75% identity to the amino acid sequence;

(2-10) the amino acid sequence as shown in SEQ ID NO.9, or an amino acid sequence having at least 75% identity to the amino acid sequence; and, the amino acid sequence as shown in SEQ ID NO.77, or an amino acid sequence having at least 75% identity to the amino acid sequence;

(2-11) the amino acid sequence as shown in SEQ ID NO.9, or an amino acid sequence having at least 75% identity to the amino acid sequence; and, the amino acid sequence as shown in SEQ ID NO.78, or an amino acid sequence having at least 75% identity to the amino acid sequence;

(2-12) the amino acid sequence as shown in SEQ ID NO.9, or an amino acid sequence having at least 75% identity to the amino acid sequence; and, the amino acid sequence as shown in SEQ ID NO.79, or an amino acid sequence having at least 75% identity to the amino acid sequence;

(2-13) the amino acid sequence as shown in SEQ ID NO.9, or an amino acid sequence having at least 75% identity to the amino acid sequence; and, the amino acid sequence as shown in SEQ ID NO.80, or an amino acid sequence having at least 75% identity to the amino acid sequence;

(2-14) the amino acid sequence as shown in SEQ ID NO.9, or an amino acid sequence having at least 75% identity to the amino acid sequence; and, the amino acid sequence as shown in SEQ ID NO.81, or an amino acid sequence having at least 75% identity to the amino acid sequence;

(2-15) the amino acid sequence as shown in SEQ ID NO.49, or an amino acid sequence having at least 75% identity to the amino acid sequence; and, the amino acid sequence as shown in SEQ ID NO.10, or an amino acid sequence having at least 75% identity to the amino acid sequence;

(2-16) the amino acid sequence as shown in SEQ ID NO.50, or an amino acid sequence having at least 75% identity to the amino acid sequence; and, the amino acid sequence as shown in SEQ ID NO.10, or an amino acid sequence having at least 75% identity to the amino acid sequence;

(2-17) the amino acid sequence as shown in SEQ ID NO.49, or an amino acid sequence having at least 75% identity to the amino acid sequence; and, the amino acid sequence as shown in SEQ ID NO.75, or an amino acid sequence having at least 75% identity to the amino acid sequence;

(2-18) the amino acid sequence as shown in SEQ ID NO.49, or an amino acid sequence having at least 75% identity to the amino acid sequence; and, the amino acid sequence as shown in SEQ ID NO.82, or an amino acid sequence having at least 75% identity to the amino acid sequence;

(2-19) the amino acid sequence as shown in SEQ ID NO.49, or an amino acid sequence having at least 75% identity to the amino acid sequence; and, the amino acid sequence as shown in SEQ ID NO.83, or an amino acid sequence having at least 75% identity to the amino acid sequence;

(2-20) the amino acid sequence as shown in SEQ ID NO.49, or an amino acid sequence having at least 75% identity to the amino acid sequence; and, the amino acid sequence as shown in SEQ ID NO.77, or an amino acid sequence having at least 75% identity to the amino acid sequence;

(2-21) the amino acid sequence as shown in SEQ ID NO.49, or an amino acid sequence having at least 75% identity to the amino acid sequence; and, the amino acid sequence as shown in SEQ ID NO.79, or an amino acid sequence having at least 75% identity to the amino acid sequence;

(2-22) the amino acid sequence as shown in SEQ ID NO.50, or an amino acid sequence having at least 75% identity to the amino acid sequence; and, the amino acid sequence as shown in SEQ ID NO.77, or an amino acid sequence having at least 75% identity to the amino acid sequence;

(2-23) the amino acid sequence as shown in SEQ ID NO.50, or an amino acid sequence having at least 75% identity to the amino acid sequence; and, the amino acid sequence as shown in SEQ ID NO.79, or an amino acid sequence having at least 75% identity to the amino acid sequence;

(2-24) the amino acid sequence as shown in SEQ ID NO.53, or an amino acid sequence having at least 75% identity to the amino acid sequence; and, the amino acid sequence as shown in SEQ ID NO.77, or an amino acid sequence having at least 75% identity to the amino acid sequence;

(2-25) the amino acid sequence as shown in SEQ ID NO.9, or an amino acid sequence having at least 75% identity to the amino acid sequence; and, the amino acid sequence as shown in SEQ ID NO.84, or an amino acid sequence having at least 75% identity to the amino acid sequence;

(2-26) the amino acid sequence as shown in SEQ ID NO.9, or an amino acid sequence having at least 75% identity to the amino acid sequence; and, the amino acid sequence as shown in SEQ ID NO.85, or an amino acid sequence having at least 75% identity to the amino acid sequence;

(2-27) the amino acid sequence as shown in SEQ ID NO.51, or an amino acid sequence having at least 75% identity to the amino acid sequence; and, the amino acid sequence as shown in SEQ ID NO.77, or an amino acid sequence having at least 75% identity to the amino acid sequence;

(2-28) the amino acid sequence as shown in SEQ ID NO.52, or an amino acid sequence having at least 75% identity to the amino acid sequence; and, the amino acid sequence as shown in SEQ ID NO.77, or an amino acid sequence having at least 75% identity to the amino acid sequence;

(2-29) the amino acid sequence as shown in SEQ ID NO.54, or an amino acid sequence having at least 75% identity to the amino acid sequence; and, the amino acid sequence as shown in SEQ ID NO.77, or an amino acid sequence having at least 75% identity to the amino acid sequence;

(2-30) the amino acid sequence as shown in SEQ ID NO.9, or an amino acid sequence having at least 75% identity to the amino acid sequence; and, the amino acid sequence as shown in SEQ ID NO.86, or an amino acid sequence having at least 75% identity to the amino acid sequence;

(2-31) the amino acid sequence as shown in SEQ ID NO.52, or an amino acid sequence having at least 75% identity to the amino acid sequence; and, the amino acid sequence as shown in SEQ ID NO.86, or an amino acid sequence having at least 75% identity to the amino acid sequence;

(2-32) the amino acid sequence as shown in SEQ ID NO.55, or an amino acid sequence having at least 75% identity to the amino acid sequence; and, the amino acid sequence as shown in SEQ ID NO.87, or an amino acid sequence having at least 75% identity to the amino acid sequence;

(2-33) the amino acid sequence as shown in SEQ ID NO.56, or an amino acid sequence having at least 75% identity to the amino acid sequence; and, the amino acid sequence as shown in SEQ ID NO.87, or an amino acid sequence having at least 75% identity to the amino acid sequence;

(2-34) the amino acid sequence as shown in SEQ ID NO.57, or an amino acid sequence having at least 75% identity to the amino acid sequence; and, the amino acid sequence as shown in SEQ ID NO.87, or an amino acid sequence having at least 75% identity to the amino acid sequence;

(2-35) the amino acid sequence as shown in SEQ ID NO.51, or an amino acid sequence having at least 75% identity to the amino acid sequence; and, the amino acid sequence as shown in SEQ ID NO.87, or an amino acid sequence having at least 75% identity to the amino acid sequence;

(2-36) the amino acid sequence as shown in SEQ ID NO.54, or an amino acid sequence having at least 75% identity to the amino acid sequence; and, the amino acid sequence as shown in SEQ ID NO.87, or an amino acid sequence having at least 75% identity to the amino acid sequence;

(2-37) the amino acid sequence as shown in SEQ ID NO.58, or an amino acid sequence having at least 75% identity to the amino acid sequence; and, the amino acid sequence as shown in SEQ ID NO.87, or an amino acid sequence having at least 75% identity to the amino acid sequence;

(2-38) the amino acid sequence as shown in SEQ ID NO.59, or an amino acid sequence having at least 75% identity to the amino acid sequence; and, the amino acid sequence as shown in SEQ ID NO.87, or an amino acid sequence having at least 75% identity to the amino acid sequence;

(2-39) the amino acid sequence as shown in SEQ ID NO.60, or an amino acid sequence having at least 75% identity to the amino acid sequence; and, the amino acid sequence as shown in SEQ ID NO.87, or an amino acid sequence having at least 75% identity to the amino acid sequence;

(2-40) the amino acid sequence as shown in SEQ ID NO.61, or an amino acid sequence having at least 75% identity to the amino acid sequence; and, the amino acid sequence as shown in SEQ ID NO.87, or an amino acid sequence having at least 75% identity to the amino acid sequence;

(2-41) the amino acid sequence as shown in SEQ ID NO.62, or an amino acid sequence having at least 75% identity to the amino acid sequence; and, the amino acid sequence as shown in SEQ ID NO.87, or an amino acid sequence having at least 75% identity to the amino acid sequence;

(2-42) the amino acid sequence as shown in SEQ ID NO.63, or an amino acid sequence having at least 75% identity to the amino acid sequence; and, the amino acid sequence as shown in SEQ ID NO.87, or an amino acid sequence having at least 75% identity to the amino acid sequence;

(2-43) the amino acid sequence as shown in SEQ ID NO.64, or an amino acid sequence having at least 75% identity to the amino acid sequence; and, the amino acid sequence as shown in SEQ ID NO.87, or an amino acid sequence having at least 75% identity to the amino acid sequence;

(2-44) the amino acid sequence as shown in SEQ ID NO.65, or an amino acid sequence having at least 75% identity to the amino acid sequence; and, the amino acid sequence as shown in SEQ ID NO.87, or an amino acid sequence having at least 75% identity to the amino acid sequence;

(2-45) the amino acid sequence as shown in SEQ ID NO.66, or an amino acid sequence having at least 75% identity to the amino acid sequence; and, the amino acid sequence as shown in SEQ ID NO.87, or an amino acid sequence having at least 75% identity to the amino acid sequence;

(2-46) the amino acid sequence as shown in SEQ ID NO.67, or an amino acid sequence having at least 75% identity to the amino acid sequence; and, the amino acid sequence as shown in SEQ ID NO.87, or an amino acid sequence having at least 75% identity to the amino acid sequence; and

(2-47) the amino acid sequence as shown in SEQ ID NO.68, or an amino acid sequence having at least 75% identity to the amino acid sequence; and, the amino acid sequence as shown in SEQ ID NO.87, or an amino acid sequence having at least 75% identity to the amino acid sequence.

4. The antibody or fragment thereof according to any one of claims 1 to 3, **characterized in that** the antibody or fragment thereof is an anti-IL-11 antibody or antigen binding fragment thereof.

5. The antibody or fragment thereof according to any one of claims 1 to 3, **characterized in that** the antibody is a murine antibody, a rabbit antibody, a human antibody, a chimeric antibody or a fully or partially humanized antibody; or a derivatized antibody.

6. The antibody or fragment thereof according to any one of claims 1 to 3, **characterized in that** the antigen binding fragment is a fragment of the antibody in any form, e.g., scFv, BsFv, dsFv, $(dsFv)_2$, Fab, Fab', $F(ab')_2$, or Fv.

7. The antibody or fragment thereof according to any one of claims 1 to 6, **characterized in that** the antibody is a monoclonal antibody, which preferably comprises murine or human heavy chain constant region and light chain constant region.

8. A nucleic acid molecule comprising a nucleotide sequence encoding the heavy chain CDRs, the light chain CDRs, the heavy chain variable region, the light chain variable region, the heavy chain and/or the light chain comprised in the antibody or fragment thereof according to any one of claims 1 to 7.

9. A vector comprising the nucleic acid molecule according to claim 8.

10. A host cell comprising the nucleic acid molecule according to claim 8 or the vector according to claim 9.

11. A composition comprising the antibody or fragment thereof according to any one of claims 1 to 7, the nucleic acid molecule according to claim 8, the vector according to claim 9, and/or the host cell according to claim 10, and optionally a pharmaceutically acceptable accessory material.

12. Use of the antibody or fragment thereof according to any one of claims 1 to 7, the nucleic acid molecule according to claim 8, the vector according to claim 9, the host cell according to claim 10, and/or the composition according to claim 11 in the manufacture of a medicament for preventing, treating, and/or ameliorating a disease or disorder associated with the expression (including overexpression) of IL-11.

13. The use according to claim 12, **characterized in that** the disease or disorder is an inflammatory disease or a tumor, e.g., liver injury, pulmonary fibrosis and colon cancer.

14. A method for preventing, treating and/or ameliorating a disease or disorder associated with the expression (including overexpression) of IL-11, comprising administering to a subject in need thereof the antibody or fragment thereof according to any one of claims 1 to 7, the nucleic acid molecule according to claim 8, the vector according to claim 9, the host cell according to claim 10, and/or the composition according to claim 11.

15. The method according to claim 14, **characterized in that** the disease or disorder is an inflammatory disease or a tumor, e.g., liver injury, pulmonary fibrosis and colon cancer.

16. The method according to claim 14 or 15, **characterized in that** the subject is a mammal; preferably, the subject is a human.

FIG. 1

Murine monoclonal antibodies

FIG. 2

**FIG. 3**

**FIG. 4**

**4a**

**4b**

# FIG. 5

**5a**

**5b**

## INTERNATIONAL SEARCH REPORT

| International application No. |
|---|
| **PCT/CN2023/073670** |

**A.    CLASSIFICATION OF SUBJECT MATTER**

C07K16/24(2006.01)i;A61K47/68(2017.01)i;A61P35/00(2006.01)i;A61P29/00(2006.01)i

According to International Patent Classification (IPC) or to both national classification and IPC

**B.    FIELDS SEARCHED**

Minimum documentation searched (classification system followed by classification symbols)

IPC: C07K A61K A61P

Documentation searched other than minimum documentation to the extent that such documents are included in the fields searched

Electronic data base consulted during the international search (name of data base and, where practicable, search terms used)

CNTXT; ENTXT; ENTXTC; WPABS; WPABSC; DWPI; CJFD; CNKI; ISI Web of Science; 中国专利生物序列检索系统, China Patent Biological Sequence Search System; GenBank; STN: 迈威(上海)生物科技股份有限公司, 曾大地, 白介素-11, 抗体, 序列1-162, IL-11, antibody

**C.    DOCUMENTS CONSIDERED TO BE RELEVANT**

| Category* | Citation of document, with indication, where appropriate, of the relevant passages | Relevant to claim No. |
|---|---|---|
| A | CN 113980129 A (DONGDA BIOTECHNOLOGY (SUZHOU) CO., LTD.) 28 January 2022 (2022-01-28)<br>claims 1-10 | 1-13 |
| A | CN 113056481 A (SINGAPORE HEALTH SERVICES PTE. LTD. et al.) 29 June 2021 (2021-06-29)<br>claims 1-25 | 1-13 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 May 2023** | **18 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/073670**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

          ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/073670** |

---

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **14-16**
   because they relate to subject matter not required to be searched by this Authority, namely:

   The subject matter of claims 14-16 is a method for prevention, treatment and/or amelioration of diseases or conditions, which relates to methods for treatment of the living human or animal body, and belongs to the subject matter for which a search is not required by the International Searching Authority as defined in PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/073670**

| Patent document cited in search report | | | Publication date (day/month/year) | Patent family member(s) | | | Publication date (day/month/year) |
|---|---|---|---|---|---|---|---|
| CN | 113980129 | A | 28 January 2022 | None | | | |
| CN | 113056481 | A | 29 June 2021 | EP | 3807314 | A1 | 21 April 2021 |
| | | | | KR | 20210031690 | A | 22 March 2021 |
| | | | | BR | 112020025443 | A2 | 16 March 2021 |
| | | | | US | 2020031918 | A1 | 30 January 2020 |
| | | | | US | 11084874 | B2 | 10 August 2021 |
| | | | | JP | 2021535733 | A | 23 December 2021 |
| | | | | AU | 2019286795 | A1 | 28 January 2021 |
| | | | | US | 2021230266 | A1 | 29 July 2021 |
| | | | | WO | 2019238882 | A1 | 19 December 2019 |
| | | | | CA | 3102483 | A1 | 19 December 2019 |
| | | | | PH | 12020552232 | A1 | 28 June 2021 |
| | | | | TW | 202003560 | A | 16 January 2020 |
| | | | | SG | 11202011782 | A1 | 31 December 2020 |

Form PCT/ISA/210 (patent family annex) (July 2022)

**REFERENCES CITED IN THE DESCRIPTION**

**Patent documents cited in the description**

- CN 202210112310
- WO 2019238882 A1
- WO 2019062755 A1